# EUROPÄISCHE PATENTSCHRIFT

(11) **EP 4 349 333 B1**
(45) Veröffentlichungstag und Bekanntmachung des Hinweises auf die Patenterteilung: **30.10.2024**
(21) Anmeldenummer: 23188553.4
(22) Anmeldetag: 28.07.2023
(51) Int. Cl.: A61K 31/44, A61P 25/00, A61P 31/14

(54) **VERWENDUNG VON AMINOPYRIDIN, INSBESONDERE AMIFAMPRIDIN, BEI DER BEHANDLUNG EINER VIRAL ASSOZIIERTEN FATIGUE**
USE OF AMINOPYRIDINE, IN PARTICULAR AMIRAMPRIDINE, IN THE TREATMENT OF VIRALLY ASSOCIATED FATIGUE
UTILISATION D'AMINOPYRIDINE, EN PARTICULIER D'AMIFAMPRIDINE, DANS LE TRAITEMENT DE LA FATIGUE ASSOCIÉE À DES VIRUS

(30) Priorität: 07.10.2022 DE 102022126035
(43) Veröffentlichungstag der Anmeldung: 10.04.2024
(73) Patentinhaber: Böhmeke, Ulrike, 45897 Gelsenkirchen (DE)
(72) Erfinder: BÖHMEKE, Thomas, 45897 Gelsenkirchen (DE); BÖHMEKE, Ulrike, 45897 Gelsenkirchen (DE)
(74) Vertreter: Bendele, Tanja

(56) Entgegenhaltungen:
- THOMAS BOEHMEKE: "Reduced Fatigue Symptoms in the Post-COVID Syndrome With Amifampridine: A Collective Casuistry With Double-Blind Discontinuation Trials", CUREUS, 25 January 2024 (2024-01-25), US, XP093155121, ISSN: 2168-8184, Retrieved from the Internet <URL:https://www.ncbi.nlm.nih.gov/pmc/articles/PMC10893954/pdf/cureus-0016-00000052935.pdf> DOI: 10.7759/cureus.52935
- SHEEAN G. ET AL: "An open-labelled clinical and electrophysiological study of 3,4 diaminopyridine in the treatment of fatigue in multiple sclerosis", BRAIN, vol. 121, no. 5, 1 May 1998 (1998-05-01), pages 967 - 975, XP093036446, DOI: 10.1093/brain/121.5.967
- JOLI JIAN ET AL: "Post-COVID-19 fatigue: A systematic review", FRONTIERS IN PSYCHIATRY, vol. 13, 11 August 2022 (2022-08-11), XP093112425, ISSN: 1664-0640, DOI: 10.3389/fpsyt.2022.947973

## Beschreibung

Die vorliegende Erfindung betrifft Pyridin-3,4-diamin, eines physiologisch verträglichen Salzes, Solvates, Hydrates, Co-Kristalls, Polymorphe dieser und/oder Gemische enthaltend zwei der genannten Verbindungen davon zur Verwendung als Medikament bei der Prophylaxe und/oder Behandlung einer Fatigue, welche mit einer viralen Infektion durch ss(+)RNA-Viren der Familie Coronaviridae, vorzugsweise durch SARS-CoV-2, assoziiert ist. Insbesondere zur Behandlung von Fatigue im Zusammenhang mit einer "Post-Covid-Phase" und dem "Long-COVID-19-Syndrom" sowie vorzugsweise in Kombination mit Erkrankungen, wie einer neuroimmunologischen Erkrankung und/oder eines chronischen Fatigue Syndroms. Gleichfalls Gegenstand der Erfindung ist Pyridin-3,4-diamin, eines physiologisch verträglichen Salzes, Solvates, Hydrates, Co-Kristalls, Polymorphs dieser und/oder Gemische enthaltend mindestens zwei der Verbindungen zur Verwendung zur Prophylaxe und/oder Behandlung einer Fatigue, welche mit einer viralen Infektion, die durch ss(+)RNA Viren der Familie Coronaviridae ausgelöst ist assoziiert ist und optional in Kombination zur Prophylaxe und/oder Behandlung einer Fatigue welche assoziiert ist mit MSC und/oder LEMS und/oder anderen Infektionserkrankungen, wie dem Pfeifferschem Drüsenfieber.

Es ist bekannt, dass neben somatischen Veränderungen wie Herzinsuffizienz, eingeschränkter Lungenfunktion nach Corona bedingter Pneumonie sowie Komplikationen Infolge venösen oder arteriellen Thrombosen, die Fatigue-Symptomatik nach Covid-Infektion eine besondere Herausforderung darstellt. Die bisherige Forschung fokussiert sich auf immunmodulatorische Therapieformen wie monoklonale Antikörper unter der Annahme einer immunologisch getriggerten Sekundärkomplikation. Eindeutige wissenschaftliche Ergebnisse liegen trotz forcierter Forschungen bislang nicht vor.

Das Fatigue-Syndrom bezeichnet ein Gefühl von anhaltender Müdigkeit, Erschöpfung und Antriebslosigkeit. Es beeinträchtigt das Leben der Betroffenen nachhaltig und lässt sich auch durch viel Schlaf nicht beseitigen. Anders als die eigenständige und seltene Erkrankung des Chronischen Fatigue-Syndroms ist das Fatigue-Syndrom auch eine Begleiterscheinung chronischer Erkrankungen wie Krebs, Rheuma und HIV/Aids oder Folge außergewöhnlicher Belastungen wie Chemotherapien. Darüber hinaus ist die Fatigue-Symptomatik auch bekannt im Rahmen von Allgemeinerkrankungen wie Anämie, Störungen der Schilddrüsenfunktion, kardialen Erkrankungen sowie Tumorleiden, aber bei auch neuromuskulären Erkrankungen wie Multipler Sklerose (MS), Myasthenia gravis (MG) sowie dem Lambert-Eaton-Myasthenie-Syndrom (LEMS). Bei vielen Patienten mit diesen Erkrankungen steht symptomatisch die Fatigue-Symptomatik im Vordergrund, die eingeschränkte Beweglichkeit infolge muskulärer Defizite wird nicht selten als nachrangig empfunden. Für das LEMS existiert eine spezifische Therapie mit Aminopyridin und zeigt im klinischen Alltag bis zu einer Tagesmaximaldosis von 60 mg keine Einschränkungen der Leber- und Nierenfunktion.

Fatigue ist somit eine krankhafte, unverhältnismäßige und durch Schlaf nicht zu beseitigende Erschöpfung, die sowohl körperlicher als auch geistiger und/oder seelischer Art sein kann. Diese Erschöpfung geht nicht auf eine vorausgegangene Anstrengung zurück. Sie ist damit von der physiologischen Müdigkeit klar abzutrennen. Die Fatigue führt zu einer verminderten zerebralen Durchblutung und ineffektiven Sauerstoffverwertung und dadurch zum Versagen der Leistungsfähigkeit und Erholungsfähigkeit. Gleichzeitig tritt eine adrenerge Hyperstimulation mit Tachykardie, Hypervigilanz und ineffektiver Ausgrenzung sensorischer Reize auf.

Der Schweregrad kann mit Fragebögen zur allgemeinen Funktionseinschränkung (Bell-Score), zur Lebensqualität (z. B. EQ5D, PedsQL, SF-36) und zu einzelnen Symptomen (Fatigue Severity Scale [FSS] sowie Chalder Fatigue Scale) erfasst werden.

Nun wurde im Zuge der COVID-19-Pandemie festgestellt, dass ein Teil der Fälle von Long-Covid und Post-COVID-19-Syndrom - auch im Kindes- und Jugendalter - klinisch als Myalgische Enzephalomyelitis/chronisches Fatigue-Syndrom (ME/CFS) verläuft bzw. diagnostiziert wird.

Die Fatigue-Symptomatik ist bislang nur klinisch fassbar, eindeutige und belastbare Laborparameter sind bisher nicht beschrieben. Fatigue äußert sich durch ein erhöhtes Schlaf- und Ruhebedürfnis, wobei eine fehlende Erholung auch nach mehreren Stunden Schlaf tagsüber bei gesteigerter Dauer des nächtlichen Schlafes typisch ist. Darüber hinaus besteht häufig eine deutlich eingeschränkte Belastbarkeit, typischerweise ist eine Etage Treppensteigen nur mit Mühe möglich, ohne dass eine korrelierende kardiale, pulmonale oder anderweitige internistische Problematik nachgewiesen werden kann. Eine manifeste Fatigue bedeutet eine gravierende Einschränkung nicht nur zur Bewältigung der täglichen Verrichtungen sondern auch für die Berufsausübung.

Sheean G. et al, "An open-labelled clinical and electrophysical study of 3,4 diaminopyridine in the treatment of fatigue in multiple sclerosis", Brain, Bd. 121, Nr. 5, Mai 1998 , S. 967-975 offenbart eine Studie zur Behandlung einer MS assoziierten Fatigue. Joli Jilan et al "Post-COVID-19 fatigue: A systematic review" offenbart die Gabe von Qingjin Yiqi Granulat im vorgenannten Zusammenhang.

Die Aufgabe der Erfindung ist es, ein Mittel bereitzustellen, dass geeignet ist, die durch eine Covid-Infektion hervorgerufenen Symptome, wie das Fatigue-Symptom, zu behandeln und zu reduzieren. Insbesondere ist es Aufgabe der vorliegenden Erfindung, eine neue Therapie zur Behandlung von Patienten, insbesondere von Kindern und Erwachsenen, die an Long-Covid oder Post-COVID-19-Syndrom leiden, bereitzustellen. Es soll eine mögliche Prophylaxe, Prävention und/oder Behandlung bereitgestellt werden, welche die Fatigue vorbeugt oder reduziert, den Bell-score verbessert oder gänzlich aufhebt und vorzugsweise einen Score von bis zu 100 erzielt. Gleichfalls soll eine effektive Wirkdosis oder Effektivdosis (ED), für das Mittel aufgefunden werden. Eine effektive Wirkdosis ist die Dosierung des freien Amins das optional als Salz, Co-Kristall etc. vorliegen kann.

Gelöst wird die Aufgabe mit Pyridin-3,4-diamin zur Verwendung zur Prophylaxe und/oder Behandlung einer Fatigue nach Anspruch 1.

Die Erfindung wird durch die Ansprüche definiert. Gegenstände, die nicht durch den Schutzumfang der Ansprüche umfasst sind, werden nur zu Informationszwecken aufgeführt.

Verweise auf Behandlungsmethoden durch Therapie in dieser Beschreibung sind als Verweise auf Verbindungen, pharmazeutische Zusammensetzungen und Arzneimittel der vorliegenden Erfindung zur Verwendung in diesen Methoden zu verstehen.

Überraschend wurde gefunden, dass ein 3,4-Diaminopyridin zur Verwendung zur Prophylaxe und/oder Behandlung von einer Fatigue, welche mit einer viralen Infektion assoziiert, insbesondere ausgelöst ist nach Anspruch 1 eingesetzt werden kann. Und optional zur Verwendung zur Prophylaxe und/oder Behandlung einer komplexen neuroimmunologischen Erkrankung umfassend Myalgische Enzephalomyelitis und/oder optional Chronische Fatigue Syndrom (ME/CFS), bevorzugt eines postinfektiösen Erschöpfungssyndroms eingesetzt werden kann. Besonders bevorzugt mit einer Effektivdosis in Bezug auf den Wirkstoff 3,4,-Diaminopyridin, von ein bis drei Dosen von 5 mg pro Tag. Eine bevorzugte Gesamtdosis kann bei 5 mg bis 15 mg pro Tag liegen. Je nach Patient wird eine Einzeldosis von 5 mg pro Tag vor dem Schlafengehen, insbesondere am Abend eingenommen. Als am Abend wird eine Uhrzeit ab ca. 18:00 angesehen und vor dem Schlafengehen wird als ca. 0 bis 2 Stunden vor dem Schlafengehen angesehen, insbesondere 0 bis 60 Minuten vor dem Schlafengehen, besonders bevorzugt 0 bis 45 min, 30 min, besonders bevorzugt 15 Minuten vor dem Schlafengehen verstanden.

Pyridin-3,4-diamin (IUPAC) oder auch 3,4-Diaminopyridin, 3,4-DAP (CAS-Nr: 54-96-6, Amifampridin) der Formeln: 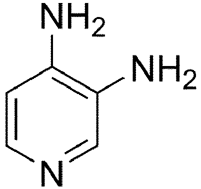 und seine Derivate (nicht erfindungsgemäß) sind bekannt und konkrete Verbindungen zur Behandlung zugelassen Pyridin-3,4-diamin verhindert durch reversibles Blockieren, dass Kaliumionen die Nervenzellen verlassen. Dadurch wird die Depolarisation am präsynaptischen Ende der Nervenzellen verlängert. Die verlängerte Depolarisation hat wiederum einen Einfluss auf Freisetzung von Acetylcholin in die Synapsen hinein. Die erhöhte Freisetzung von Acetylcholin verbessert die Muskelkontraktion. Derivate umfassen Diaminopyridin, insbesondere 3,4-Diaminopyridin bzw. Pyridin-3,4-diamin. Amifampridin kann die spannungsabhängigen Kaliumkanäle, blockieren und kann dadurch die Depolarisation der präsynaptischen Zellmembran verlängern und kann die Acetylcholinfreisetzung an den Synapsen verbessern, kann die Erregungsleitung verbessern und letztendlich die Muskelfunktion verbessern.

Aminopyridin umfasst Pyridin-3,4-diamin, 4-Aminopyridin, Diaminpyridin sowie die physiologisch verträglichen Salze, bevorzugt wird Pyridin-3,4-diamin (erfindungsgemäß) und/oder sowie 4-Aminopyridin und/oder 3-Aminopyridrin.

EP 0 159 112 offenbart Amifampridin und das Monohydrochloridsalz. US 2004/0106651 offenbart die Herstellung von Phosphat- und Tartratsalz von Amifampridin und deren Verwendung zur Behandlung von Botulismus, Myasthenie, myasthenischen Syndromen und Müdigkeit im Zusammenhang mit einer neurologischen Erkrankung, z. B. Multiple Sklerose oder amyotrophe Lateralsklerose (ALS). EP3696169A1 offenbart die Herstellung eines Phosphatsalzes von Amifampridin.

In einem ersten Aspekt betrifft die vorliegende Erfindung eine Verwendung von Pyridin-3,4-diamin und/oder eines physiologisch verträglichen Salzes, Solvates, Hydrates, Co-Kristalls, Polymorphe dieser und/oder Gemische enthaltend zwei der genannten Verbindungen bei der Prophylaxe, Prävention und/oder Behandlung einer Fatigue, welche mit einer viralen Infektion gemäß Anspruch 1 assoziiert ist.

Vorzugsweise ist die Fatigue im Sinne der Erfindung, eine wie hier beschrieben. Insbesondere ist es eine Fatigue, die analog nach ICD-Code G93.3 als chronisches Müdigkeitssyndrom [Chronic fatigue syndrome] diagnostiziert werden könnte. Eine Fatigue, welche mit einer viralen Infektion assoziiert ist, ist der Diagnostik zum ICD-Code G93.3 ähnlich oder vergleichbar. Jedoch ist die Ursache in der viralen Infektion begründet.

Im Sinne der Erfindung wird die virale Infektion durch ss(+)RNA-Viren der Familie Coronaviridae umfassend das Virus SARS-CoV-1, SARS-CoV-2, MERS-CoV, HCoV-HKU1, HCoV-OC43, HCoV-NL63 und/oder HCoV-229E, oder durch ein Virus der Familie der Herpesviridae (nicht erfindungsgemäß) umfassend Epstein-Barr-Virus ausgelöst.

In einem bevorzugten Aspekt der Verwendung im Sinne der Erfindung, ist die virale Infektion eine SARS-CoV-2 Infektion.

In einem vierten Aspekt der Verwendung im Sinne der vorliegenden Erfindung, ist die Fatigue eine postinfektiöse Fatigue, vorzugsweise welche nach Abklingen der viralen Infektion auftritt, vorzugsweise nach Feststellen eines Ct-Wertes von größer gleich 15. Vorzugsweise größer gleich 20, größer gleich 25, größer gleich 30, größer gleich 35 oder mehr. Gleichfalls Gegenstand der Erfindung ist die Verwendung im Sinne der Erfindung zur Verhütung, zur Vermeidung und/oder Behandlung einer komplexen neuroimmunologischen Erkrankung umfassend Myalgische Enzephalomyelitis und/oder Chronisches Fatigue Syndrom (ME/CFS), bevorzugt eines postinfektiösen Erschöpfungssyndroms.

Der Ct-Wert (cycle-threshold-Wert) gibt die Anzahl an Messzyklen an, die innerhalb einer PCR-Probe durchgeführt werden, um das Coronavirus nachweisen zu können. Je größer der Ct-Wert, umso geringer die Viruslast in der gemessenen Probe. Mit zunehmenden Ct-Wert entfernt sich der Krankheitsverlauf des Patienten aus der akuten Krankheitsphase und der Patienten geht in eine post-postinfektiöse Krankheitsphase über. Der ct-Wert alleine ist nicht immer verlässlich und es kann vorteilhaft sein die Umrechnung von Ct-/Cq-Werten in Virus-RNA-Lasten (RNA-Kopien pro Probenvolumen) durch Kalibration mit Hilfe einer standardisierten Virus-RNA-Präparation heranzuziehen.

In einem weiteren Aspekt der Verwendung im Sinne der Erfindung, bestehen die gesundheitlichen Beschwerden der akuten Krankheitsphase einer viralen, vorzugwiese SARS-CoV-2, Infektion, über größer gleich 3, größer gleich 4 Wochen fort oder sie treten neu auf. Besonders bevorzugt ist der Patient in einer sogenannten "Post-Covid-Phase", wie in den Leitlinienempfehlung des britischen National Institute for Health and Care Excellence (NICE) definiert. Demnach werden als Post-COVID-Syndrom Beschwerden bezeichnet, die noch mehr als 12 Wochen nach Beginn der SARS-CoV-2-Infektion vorhanden sind und nicht anderweitig erklärt werden können. "Long COVID" umfasst sowohl im Anschluss an eine akute COVID-19-Erkrankung 4 bis 12 Wochen nach Symptombeginn noch bestehende Symptome als auch das "Post-COVID-19-Syndrom".

Daher ist ein weiterer Aspekt der erfindungsgemäßen Verwendung, dass die Beschwerden der akuten Krankheitsphase einer viralen, vorzugwiese SARS-CoV-2, Infektion, mehr als 4, vorzugsweise mehr als 5, mehr als 6, mehr als 7, mehr als 8, mehr als 9, mehr als 10, mehr als 11 oder mehr als 12 Wochen nach Beginn der viralen, vorzugsweise SARS-CoV-2, Infektion fortbestehen. Insbesondere weist der Patient ein "Long-COVID-19-Syndrom" auf, insbesondere nach der Leitlinienempfehlung des britischen National Institute for Health and Care Excellence (NICE). Die NICE Richtlinien [NG188] wurden am 18. Dezember 2020 veröffentlicht und zuletzt am 11 November 2021 aktualisiert. "Mehr als" soll synonym zu "größer gleich" verstanden werden.

Eine akute Krankheitsphase einer viralen Infektion im Sinne der vorliegenden Erfindung umfasst Symptome wie Husten, Fieber, Schnupfen, Störung des Geruchs- und/oder Geschmackssinns, Halsschmerzen, Atemnot, Kopf- und Gliederschmerzen, Appetitlosigkeit, Gewichtsverlust, Übelkeit, Bauchschmerzen, Erbrechen, Durchfall, Konjunktivitis, Hautausschlag, Lymphknotenschwellung, Apathie und/oder Somnolenz. Die Symptome müssen nicht alle gleichzeitig und nicht alle insgesamt auftreten. Jeder Patient weist eine individuelle Symptomatik auf, die ein oder mehrere der vorgenannten Symptome gleichzeitig oder nacheinander aufweisen kann.

In einem weiteren Aspekt der erfindungsgemäßen Verwendung umfasst die Fatigue Symptome ausgewählt aus Schlafstörungen, Müdigkeit, erhöhtes Schlafbedürfnis, muskuläre Schwäche, verringerte körperliche Belastbarkeit, verringerte psychische Belastbarkeit, verringertes Erinnerungsvermögen, Denk- und Merkbeeinträchtigung, Konzentrationsverlust, erhöhtes Schmerzempfinden und/oder ein Aufmerksamkeitsdefizit und/oder Fehlregulationen von Kreislauf, Hormon- und/oder des Immunsystem umfasst. Die Symptome können auf durch Befragung ermittelte Symptome beschränkt sein, aber auch in Kombination mit biochemischen Parametern auftreten, welche zur Analyse von Kreislauf, Hormon- und/oder Immunsystem herangezogen werden. Diese Parameter sind dem Fachmann bekannt und umfassen die Messung der Hormone u.a. Melatonin, Cortisol und/oder Serotonin, die Messung von dem oberen, systolische und den unteren, diastolischen Wert, Bestimmung des Blutzuckers usw.

In einem weiteren Aspekt der erfindungsgemäßen Verwendung liegt eine Fatigue bei einem Bell-Score von kleiner gleich 50, kleiner gleich 40, kleiner gleich 30, kleiner gleich 20 vor. Zur Bestimmung des Bell-Score wird der jeweils aktuelle Zustand des Patienten relativ zum Zustand des Patienten zu einem Zeitpunkt vor der viralen Infektion festgestellt.

In einem weiteren Aspekt der erfindungsgemäßen Verwendung, insbesondere durch die Verabreichung des mindestens einen wirksamen Pyridin-3,4-diamin, eines physiologisch verträglichen Salzes, Solvates, Hydrates, Co-Kristalls, Polymorphe dieser und/oder Gemische enthaltend zwei der genannten Verbindungen wird der Schlafbedarf auf physiologische Stunden größer gleich 6 bis kleiner gleich 12 reduziert, vorzugsweise größer gleich 6, größer gleich 7, größer gleich 8 bis kleiner gleich 11 Stunden, kleiner gleich 10, kleiner gleich 9, besonders bevorzugt größer gleich 6 bis kleiner 8.

Ein weiterer Aspekt der vorliegenden Erfindung, ist eine Zusammensetzung enthaltend mindestens ein Pyridin-3,4-diamin, eines physiologisch verträglichen Salzes, Solvates, Hydrates, Co-Kristalls, Polymorphe dieser und/oder Gemische enthaltend zwei der genannten Verbindungen zur Verwendung bei der Prävention, Prophylaxe, Verhütung, Vermeidung der Ausbildung und/oder Behandlung einer Fatigue wie hierin beschrieben, welche mit einer viralen Infektion assoziiert ist, gemäß Anspruch 14. Gleichfalls offenbart ist ein Verfahren zur Behandlung, Verhütung der Ausbildung und/oder Vermeidung der Entwicklung einer komplexen neuroimmunologischen Erkrankung umfassend Myalgische Enzephalomyelitis und/oder Chronisches Fatigue Syndrom (ME/CFS), bevorzugt eines postinfektiösen Erschöpfungssyndroms.

Gleichfalls Gegenstand der Erfindung ist Pyridin-3,4-diamin, eines physiologisch verträglichen Salzes, Solvates, Hydrates, Co-Kristalls, Polymorphs dieser und/oder Gemische enthaltend mindestens zwei der Verbindungen zur Verwendung zur Prophylaxe und/oder Behandlung einer Fatigue, welche mit einer viralen Infektion, die durch ss(+)RNA Viren der Familie Coronaviridae ausgelöst ist assoziiert ist und optional in Kombination zur Prophylaxe und/oder Behandlung einer Fatigue welche assoziiert ist mit MSC und/oder LEMS und/oder anderen Infektionserkrankungen, wie dem Pfeifferschem Drüsenfieber und/oder Influenzaviren, insbesondere Influenzaviren aus der Familie der Orthomyxoviridae, welche die Krankheit Influenza auslösen können. Die Influenzaviren sind behüllte Viren mit einzelsträngigen, segmentierten RNA von negativer Polarität als Genom (+/-ssRNA).

Die folgenden Gattungen werden den Influenzaviren zugeordnet: Alpha-, Beta-, Gamma- und Deltainfluenzavirus. Die Spezies umfassen vorzugsweise Influenza A-Virus (FLUAV) bis Influenza D-Virus (FLUDV).

Gegenstand der Erfindung ist 3,4-Diaminopyridin bzw. Pyridin-3,4-diamin, eines physiologisch verträglichen Salzes, Solvates, Hydrates, Co-Kristalls, Polymorphe dieser und/oder Gemische enthaltend zwei der genannten Verbindungen zur Verwendung zur Prophylaxe und/oder Behandlung einer Fatigue, wie hierin beschrieben, welche mit einer viralen Infektion assoziiert ist und/oder zur Prophylaxe und/oder Behandlung einer komplexen neuroimmunologischen Erkrankung umfassend Myalgische Enzephalomyelitis und/oder Chronisches Fatigue Syndrom (ME/CFS), bevorzugt eines postinfektiösen Erschöpfungssyndroms. Bevorzugt ist die Verwendung wie hierin beschrieben. Besonders bevorzugt von größer gleich 2 mg in 24 Stunden in Bezug auf den Wirkstoff 3,4-Diaminopyridin, weiter bevorzugt von größer gleich 5 mg in 24 Stunden bis 100 mg innerhalb von 24 Stunden in Bezug auf den Wirkstoff 3,4-Diaminopyridin. Entsprechend einer besonders bevorzugten Alternative einer Verabreichung von mindestens zweimal täglich, insbesondere innerhalb von 24 Stunden, von jeweils größer gleich 2 mg, bevorzugt von jeweils 5 mg in Bezug auf den Wirkstoff. Eine besonders bevorzugte Tagesdosis innerhalb von 24 Stunden beträgt 0 bis 15 mg, insbesondere 5 mg bis 15 mg, bevorzugt zu je 5 mg Wirkstoff je Einzeldosis. Dabei ist es besonders bevorzugt ein bis drei Einzeldosen von je 1 bis 10 mg Wirkstoff, bevorzugt von 2 mg bis 7 mg, besonders bevorzugt von 5 mg plus/minus 2 mg, bevorzugt von plus minus 1 mg je Einzeldosis, als Tagesdosierung zu verwenden, insbesondere zu den offenbarten Zwecken zu verwenden. Gegenstand der Erfindung ist ein Verfahren zur Prophylaxe und/oder zur Behandlung einer Fatigue, welche mit einer viralen Infektion assoziiert und/oder einer komplexen neuroimmunologischen Erkrankung umfassend Myalgische Enzephalomyelitis und/oder Chronisches Fatigue Syndrom (ME/CFS), bevorzugt eines postinfektiösen Erschöpfungssyndrom mit 3,4-Diaminopyridin bzw. Pyridin-3,4-diamin, eines physiologisch verträglichen Salzes, Solvates, Hydrates, Co-Kristalls, Polymorphe dieser und/oder Gemische enthaltend zwei der genannten Verbindungen.

Unter postinfektiösem Erschöfpungssyndrom wird ein Erschöpfungssyndrom verstanden, das mit einer viralen Infektion, insbesondere umfassend virale Infektionen, mit einen oder mehreren der Viren SARS-CoV-1, SARS-CoV-2, MERS-CoV, HCoV-HKU1, HCoV-OC43, HCoV-NL63 und/oder HCoV-229E, oder der Familie der Herpesviridae umfassend Epstein-Barr-Virus, assoziiert ist, insbesondere durch eine virale Infektion ausgelöst und/oder verstärkt wurde.

Pharmazeutische Zusammensetzung umfassend, insbesondere einen Kaliumkanal-Blocker, bevorzugt Pyridin-3,4-diamin, insbesondere als Wirkstoff, eines physiologisch verträglichen Salzes, Solvates, Hydrates, Co-Kristalls, Polymorphe dieser und/oder Gemische enthaltend zwei der genannten Verbindungen sowie mindestens eines pharmazeutischen Hilfsstoffes zur Verwendung zur Prophylaxe und/oder Behandlung einer Fatigue wie hierin beschrieben, welche mit einer viralen Infektion assoziiert ist und/oder zur Prophylaxe und/oder Behandlung einer komplexen neuroimmunologischen Erkrankung umfassend Myalgische Enzephalomyelitis und/oder Chronisches Fatigue Syndrom (ME/CFS), bevorzugt eines postinfektiösen Erschöpfungssyndroms. Besonders bevorzugt ist eine pharmazeutische Zusammensetzung zur Verwendung nach einem der Ansprüche 1 bis 13. Pharmazeutische Zusammensetzung umfassend größer gleich 2 mg bis 15 mg in Bezug auf den Wirkstoff Pyridin-3,4-diamin, bevorzugt von 5 mg mit plus/minus 5 Gew.-% in Bezug auf den Wirkstoff Pyridin-3,4-diamin. Pharmazeutische Zusammensetzung umfassend 1 mg, größer gleich 2 mg, größer gleich 3, vorzugsweise größer gleich 5 mg bis kleiner gleich 60 mg Pyrdin-3,4-diamin. Dabei bezieht sich die Angabe in mg auf die freie Base, auf die Base im Salz, die Base im Co-Kristall etc.

Gegenstand der Erfindung ist 3,4-Diaminopyridin bzw. Pyridin-3,4-diamin, eines physiologisch verträglichen Salzes, Solvates, Hydrates, Co-Kristalls, Polymorphe dieser und/oder Gemische enthaltend zwei der genannten Verbindungen zur Verwendung zur Prophylaxe und/oder Behandlung einer Fatigue im Zusammenhang mit einer "Post-Covid-Phase" und dem "Long-COVID-19-Syndrom" sowie vorzugsweise in Kombination mit Erkrankungen, wie einer neuroimmunologischen Erkrankung und/oder eines chronischen Fatigue Syndroms. Gleichfalls Gegenstand der Erfindung ist Pyridin-3,4-diamin, eines physiologisch verträglichen Salzes, Solvates, Hydrates, Co-Kristalls, Polymorphs dieser und/oder Gemische enthaltend mindestens zwei der Verbindungen zur Verwendung zur Prophylaxe und/oder Behandlung einer Fatigue, welche mit einer viralen Infektion, die durch ss(+)RNA Viren der Familie Coronaviridae ausgelöst ist assoziiert ist und optional in Kombination zur Prophylaxe und/oder Behandlung einer Fatigue welche assoziiert ist mit MSC und/oder LEMS und/oder anderen Infektionserkrankungen, wie dem Pfeifferschem Drüsenfieber.

Als physiologisch verträgliche Salze werden Salze der Phosphorsäure, mit Phosphat(en), Phosphatester, HX, wobei HX umfasst X⁻ umfassend Chlorid, Bromid, Jodid und mit H in HX gleich H⁺, Zink-Salze, als auch Hydrate und/oder Solvate dieser Salze von Pyridin-3,4-diamin verstanden. Bevorzugt sind Dihydrohalogenide, wie Dihydrochlorid als auch die Hydrate, Solvate und/oder Gemische dieser. Phosphatester umfassen Ester der Orthophosphorsäure (H₃PO₄). Gleichfalls können als physiologisch verträgliche Salze Zink-, Erdalkali- und/oder Alkalimetallsalze von Polycarbonsäuren verwendet werden.

Als Solvate von Pyridin-3,4-diamin gelten Alkohol oder Polyalkylen, mit Alkylen mit 1 bis 4 C-Atomen, enthaltende Molekülkomplexe von Pyridin-3,4-diamin als auch der Salze von Pyrdidin-3,4-diamin. Bevorzugte Alkohole umfassen Ethanol, iso-Propanol und/oder Butanol.

Als Hydrate gelten stöchiometrische und nicht-stöchiometrische amorphe oder kristalline Molekülverbindungen umfassend Pyridin-3,4-diamin und Wasser.

Als physiologisch verträgliche Salze werden Salze mit organischen Carbonsäuren, insbesondere Hydroxy-funktionellen Carbonsäuren, Fruchtsäuren, Fettsäuren, Mono- oder Dicarbonsäuren, Tricarbonsäuren, ungesättigte Mono-, Di- und/oder Tricarbonsäuren oder Salze mit Alkali-Ionen und/oder Erdalkali-Ionen mit mindestens einem Carboxylat der vorgenannten Carbonsäuren verstanden. Geeignete Carbonsäuren umfassen gesättigte oder ungesättigte Mono-, di- oder Tri-Carbonsäuren mit 1 bis 15 C-Atomen, wie Milchsäure, Fumarsäure, Bernsteinsäure, Glutarsäure, Adipinsäure, Zitronensäure, und/oder deren Carboxylate, Salze mit Alkali- und/oder Erdalkaliionen und Gemische umfassend mindestens zwei der genannten Verbindungen.

Co-Kristalle können Vitamine, wie Nicotinamid, Folsäure und/oder Ascorbinsäure, umfassen. Weitere Vitamine mit denen Co-Kristalle hergestellt werden können umfassen Thiamin, Riboflavin, Niacin, Nicotinsäureamid, Nicotinsäure, Pantothensäure, Vitamin B6, Biotin, Folsäure, Vitamin B12, Cobalamin, Cholecalciferol, Tocopherol, Phyllochinon und/oder Menachinon oder Mischungen umfassend mindestens zwei der genannten Verbindungen. Weitere Verbindungen mit denen Co-Kristalle hergestellt werden können umfassen: Saccharin, Nicotinamid, Triestersäure, Nikotinsäureamid, 18-Kronen-6, Bernsteinsäure, Fumarsäure, Weinsäure, Apfelsäure, Malonsäure, Benzamid, Mandelsäure, Glykolsäure, Harnstoff, Nikotinsäure, 2,6-Pyridincarbonsäure, 5-Nitroisophthalsäure, 1,3,5,7-Adamantantan-Tetracarbonsäure und/oder Mischungen mindestens zwei der genannten Verbindungen, optional in Kombination mit einem HX Salz.

Die Zusammensetzung kann weitere pharmazeutische zulässige Hilfsstoffe umfassen: Füllstoffe, Zerfallsbeschleuniger, Gleitmittel, Sprengmittel, Schmiermittel, Formtrennmittel, Fließregulatoren, Lösungsmittel, Emulgatoren, Lösungsvermittler, Solubilisatoren, Netzmittel, Salzbildner, Puffer, Gelbildner, Verdickungsmittel, Filmbildner, Bindemittel, Sorptionsmittel, Süßmittel, Färbemittel, Weichmacher, Stabilisatoren, Matrixbildner, Polymere, Säuerungsmittel, Konservierungsmittel, Duftstoffe und/oder retardierende Mittel. Weitere Hilfsstoffe umfassen Trägerstoffe, Konservierungsstoffe, Antioxidantien, Stabilisatoren, Vitamine, Färbemittel, Geruchsverbesserer und Geschmacksstoffe.

Geschmacksstoffe dienen zur Überdeckung eines potentiell unangenehmen Geschmacks. Hierzu eignen sich z.B. Minze, Anis, Fenchel, Menthol, Kümmel, Medizinalhonig, Honig, Agavensaft, Zucker, Zuckerersatz sowie -austauschstoffe, Propolis und andere nach dem Stand der Technik bekannte Geschmacks- und Aromastoffe.

Ein weiterer Gegenstand der vorliegenden Erfindung ist eine Zusammensetzung enthaltend mindestens ein Pyridin-3,4-diamin, eines physiologisch verträglichen Salzes, Solvates, Hydrates, Co-Kristalls, Polymorphe dieser und/oder Gemische enthaltend zwei der genannten Verbindungen, wobei die Zusammensetzung in flüssiger, pastöser oder fester Form vorliegt.

Flüssige Formen umfassen Lösungen, Dispersionen, Suspensionen und Emulsionen enthaltend mindestens ein Aminopyridin im Sinne der Erfindung, können die üblichen Hilfsstoffe umfassen, wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1, 3-Butylglykol, Öle, Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische aus mindestens zwei der vorgenannten Stoffe. Bevorzugte Hilfsstoffe für Lösungen, Dispersionen und Suspensionen enthaltend das mindestens eine Pyridin-3,4-diamin, eines physiologisch verträglichen Salzes, Solvates, Hydrates, Co-Kristalls, Polymorphe dieser und/oder Gemische enthaltend zwei der genannten Verbindungen umfassen Dextrose, Mannitol, Tragant, Pektin, Methylcellulose, Hydroxypropylmethylcellulose (HPMC), Carboxymethylcellulose-Natrium, Polyvinylpyrrolidon, Polyvinylalkohol, Polyacrylsäure, Polyethylenglycol, Polyethylenoxid, Natriumdodecylsulfat, Natriumcetylstearylsulfat, und/oder Na-dioctylsulfosuccinat (auch K, Ca-salze) und insbesondere für Suspensionen auch Cellulose.

Im Sinne der Erfindung liegt die Zusammensetzung in einer Form vor, die zur oralen Verabreichung geeignet ist umfassend i) feste Formen, wie Tablette, Pulver, Granulat, Brausetablette, Trockensaft, Dragee, Globuli, Kapsel und Lyophylisat und ii) flüssige Formen, wie Suspension, Lösung, Dispersion, Tinktur, Konzentrat, Tee. Tabletten im Sinne der Erfindung umfassen einfache Tabletten, Lutsch-, Sublingual- und Buccaltabletten, oral dispersible Tabletten, Lösungs-, Mikro-, Kau- und Brausetabletten, Punkt-, Gerüst- und Mehrschichttabletten, Drageés und Pellets. Retardierende Eigenschaften weisen z.B. die Hilfsstoffe Ethyl- und Methylcellulose, Hydroxy-propylmethylcellulose, Celluloseacetatphthalat und Hydroxypropylmethylcellulosephthalat auf.

Im Sinne der Erfindung ist eine Tablette bevorzugt, enthaltend größer gleich 5 mg, größer gleich 10 mg an Pyridin-3,4-diamin, eines physiologisch verträglichen Salzes, Solvates, Hydrates, Co-Kristalls, Polymorphe dieser und/oder Gemische enthaltend zwei der genannten Verbindungen. Besonders bevorzugt ist ein Diaminpyridin. Die jeweilige Tablette ist vorzugsweise portionierbar.

Die Dosis, insbesondere eine Anfangsdosis, beträgt vorzugsweise 3 bis 5 mg, vorzugsweise bis zu dreimal täglich (d. h. 9 bis 15 mg pro Tag). Die Dosis kann langsam und mehrmals täglich, vorzugsweise auf viermal täglich gesteigert werden (d. h. auf bis zu 20 mg pro Tag). Die tägliche Gesamtdosis kann weiter gesteigert werden, 4 bis 5 Tage 5 mg pro Tag hinzugefügt werden, wobei eine maximal Dosis 60 mg pro Tag betragen sollte. Pro Tag bedeutet vorzugsweise innerhalb eines Zeitraumes von 24 h.

Die Verbindung hat eine verkürzte Halbwertzeit. Daher empfiehlt es sich mehrere vergleichsweise geringere Dosen über den Tag verteilt zu verabreichen oder komplexe retardierte Formulierungen zu verwenden. Mögliche Formulierungen im Sinne der Erfindung sind in der nachstehenden Tabelle 1 aufgeführt. Dabei bedeutet die Zahl 1 in der Tabelle 1, dass die jeweiligen Hilfsstoffe in der Formulierung die genannte Applikation oder technische Funktion erfüllen können.

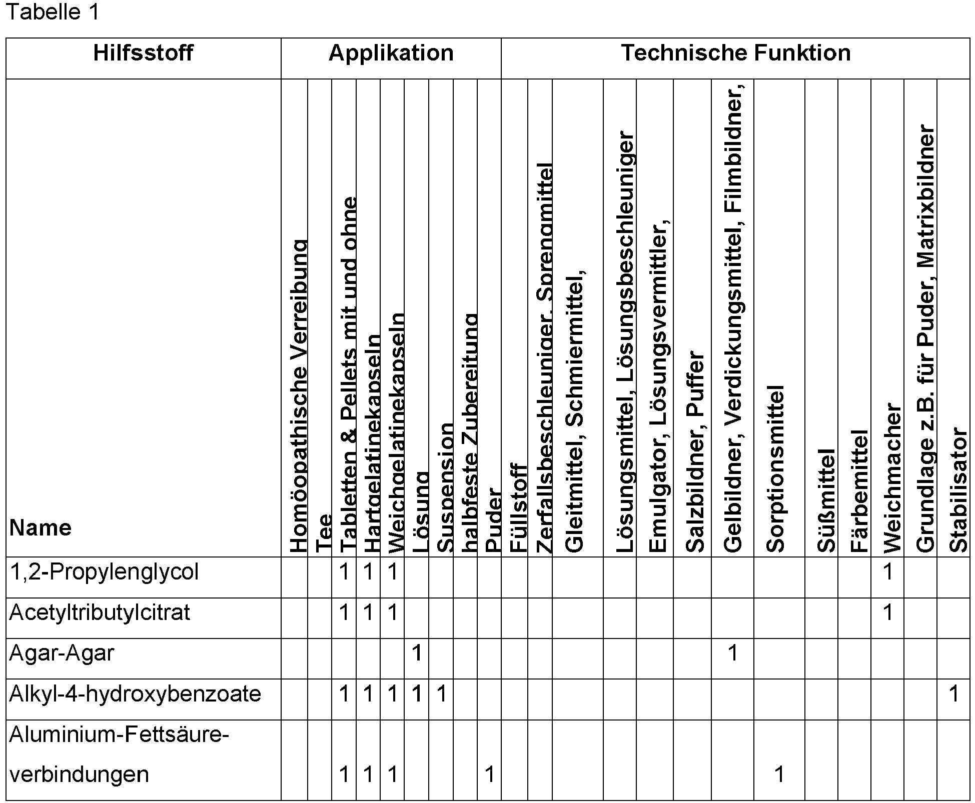

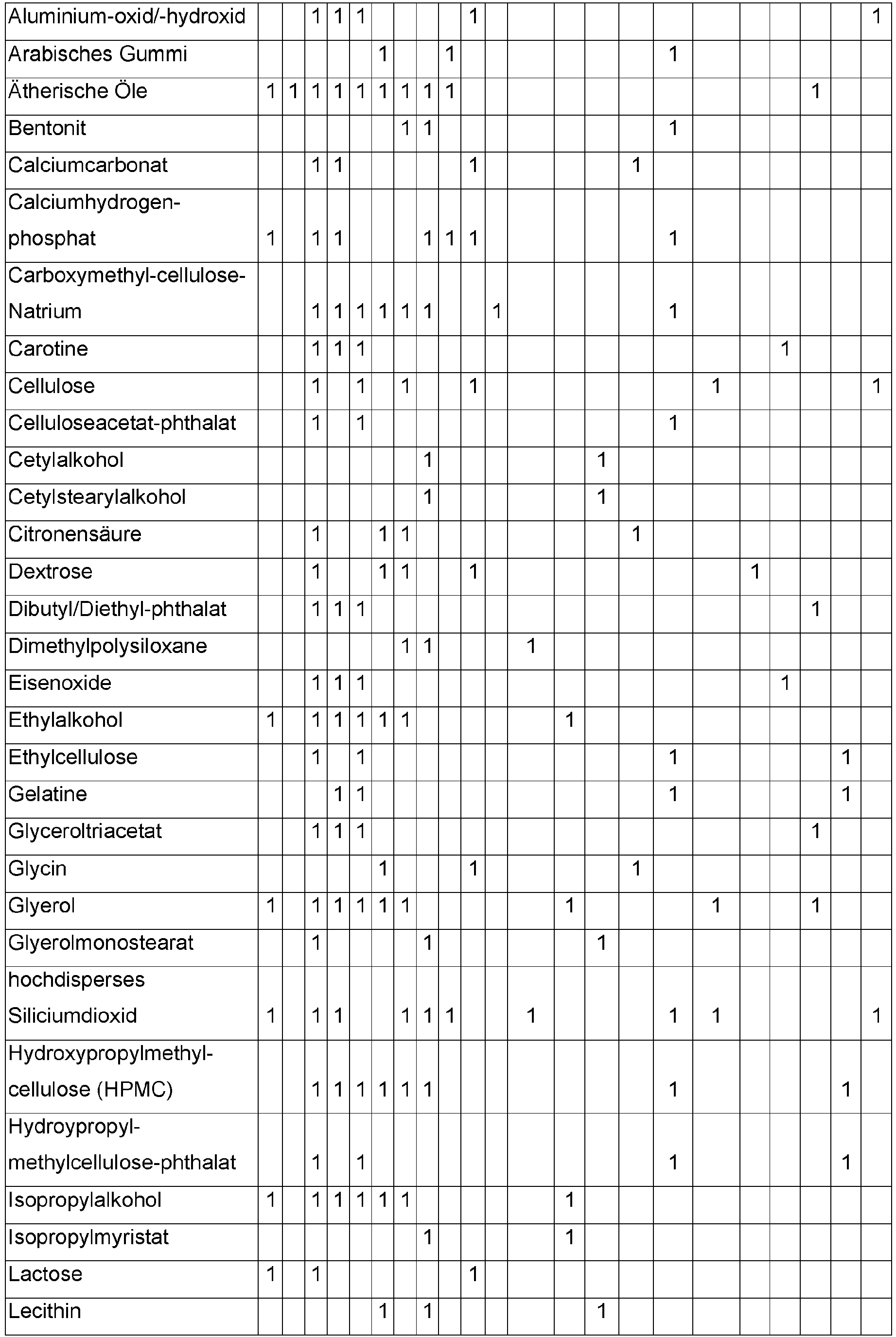

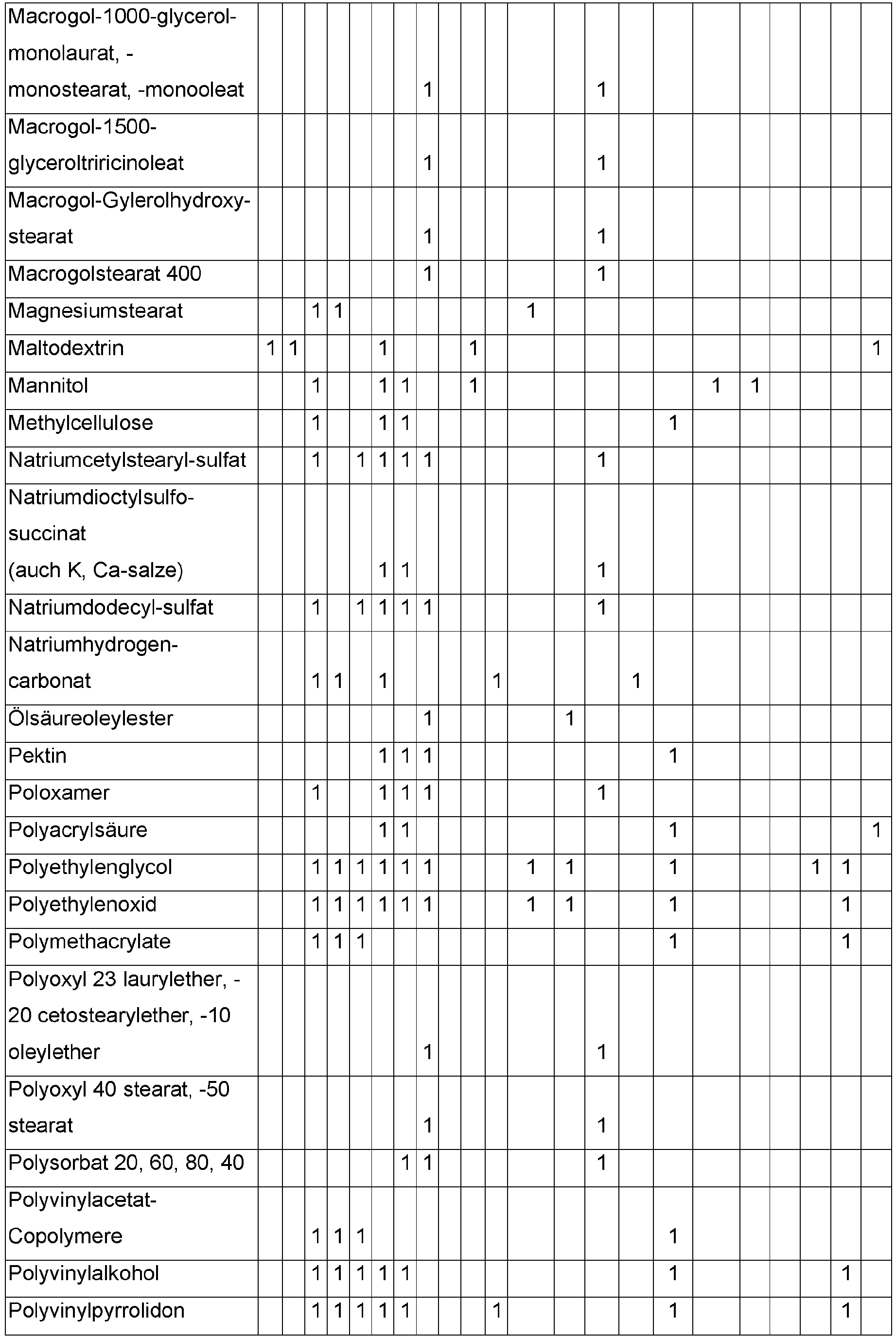

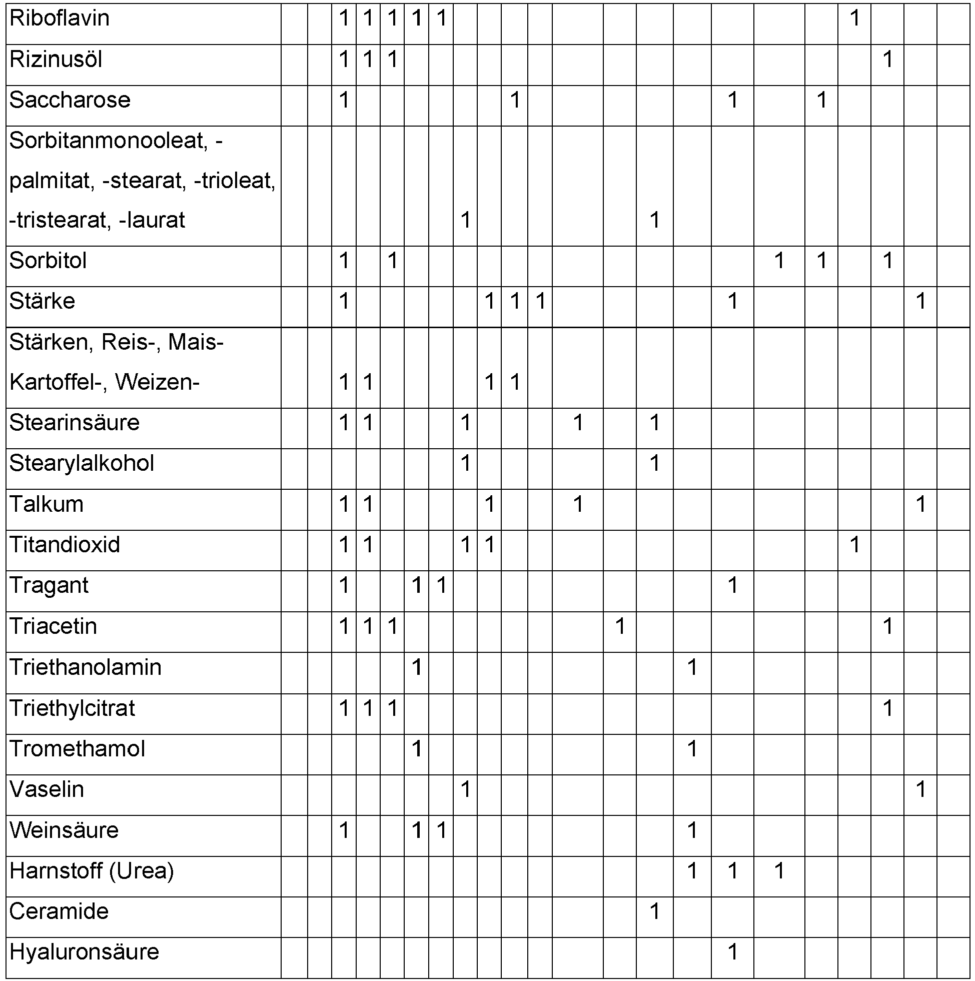

Nach einem weiteren Aspekt wird Pyridin-3,4-diamin, eines physiologisch verträglichen Salzes, Solvates, Hydrates, Co-Kristalls, Polymorphe dieser und/oder Gemische enthaltend zwei der genannten Verbindungen oder ein erfindungsgemäße Zusammensetzung mit einer Dosis von größer gleich 1 mg, größer gleich 2 mg, größer gleich 3, vorzugsweise größer gleich 5 mg bis kleiner gleich 60 mg pro Tag, besonders bevorzugt von 5 mg bis 20 mg, bevorzugt von 5 mg bis 15 mg, insbesondere pro 24 Stunden, verabreicht.

Die zulässige Tageshöchstdosis der als Arzneimittel zugelassenen Pyridin-3,4-diamin, eines physiologisch verträglichen Salzes, Solvates, Hydrates, Co-Kristalls, Polymorphe dieser und/oder Gemische enthaltend zwei der genannten Verbindungen beträgt 60 mg und die Einzeldosis darf 20 mg betragen. Eine bevorzugte Tageshöchstdosis des Wirkstoffes Pyridin-3,4-diamin beträgt 20 mg und/oder eine Einzeldosis von 5 mg bis 10 mg, besonders bevorzugt ist eine Tageshöchstdosis des Wirkstoffes Pyridin-3,4-diamin von 15 mg und/oder eine Einzeldosis 5 mg bis 10 mg, bevorzugt von 5 mg.

Daher ist ein weiterer Aspekt der erfindungsgemäßen Verwendung, dass eine maximale Tagesdosis kleiner gleich 60 mg aufweist, bevorzugt von kleiner gleich 40 mg, kleiner gleich 30 mg, kleiner gleich 20 mg, besonders bevorzugt kleiner gleich 15 mg, insbesondere verabreicht wird, vorzugsweise größer gleich eine bis kleiner gleich fünf Dosen (Einzeldosierungen) pro Tag verabreicht werden, besonders bevorzugt enthält eine Dosis größer gleich 2 mg, größer gleich 2,5 mg größer gleich 3 mg, größer gleich 2 mg bis 7 mg, bevorzugt größer gleich 2 mg bis 5 mg, weiter bevorzugt größer gleich 5 mg bis kleiner gleich 20 mg, bis kleiner gleich 18 mg, vorzugsweise bis kleiner gleich 15 mg, jeweils des mindestens einen des Wirkstoffes Pyridin-3,4-diamin, eines physiologisch verträglichen Salzes, Hydrates, Solvates, Co-Kristalls, Polymorphs dieser und/oder Mischungen von mindestens zwei der Verbindungen.

In einem weiteren Aspekt der erfindungsgemäßen Verwendung ist die Behandlung eine Langzeitbehandlung mit einer wirksamen Dosis, vorzugsweise ist die wirksame Dosis auf die Physiologie des Patienten angepasst und auf das Erreichen eines Bell-Scores von größer gleich 60, größer gleich 70, größer gleich 80, größer gleich 90 bis zu 100 und/oder ein Kraftgrad von mindestens 4 ausgerichtet. Ein Kraftgrad von 5 gilt als normal, also gesund, bei einem Kraftgrad von 3 wird von einem pflegebedürftigen Patienten gesprochen, der ans Bett gebunden ist (kann sich nicht die Nase kratzen). Bei einem Kraftgrad von 4, ist die Kraft gemindert/reduziert.

Gegenstand der Erfindung ist zudem eine einmalige Dosierung von 5 mg, insbesondere innerhalb von 1 bis 3 Stunden vor dem Schlafengehen bzw. Zubettgehen, bevorzugt innerhalb von 0 bis 60 Minuten. In einer bevorzugten Ausgestaltung ist Gegenstand der Erfindung eine einmalige Dosierung von mindestens 5 mg bis 25 mg, insbesondere innerhalb von 1 bis 3 Stunden, bevorzugt innerhalb von 0 bis 60 Minuten vor dem Schlafengehen. Die Wirkung des Amifampridins hält vorzugsweise ca. 4 Stunden an. Bevorzugt steigt der Bell-Score Wert auf mindestens 90 bis 100 und/oder das Schlafbedürfnis sinkt auf unter 11 Stunden, bevorzugt auf unter 8 Stunden. Das individuelle Schlafbedürfnis hängt auch davon ab, ob Amifampridin (Pyridin-3,4-Diamin) zur Behandlung einer Fatigue verabreicht wird, welche mit einer viralen Infektion assoziiert ist und optional einer neuroimmunologischen Erkrankung und/oder einer chronischem Fatigue Syndrom in Kombination auftritt. Besonders bevorzugt kann Amifampridin als Pharmazeutische Zusammensetzung zur Verwendung nach der Erfindung als retardiert freisetzende Zusammensetzung bereitgestellt werden. Dazu kann Amifampridin als Co-Kristall in der Formulierung zur retardierten Freisetzung vorliegen und/oder eine retardierende Zusammensetzung bereitgestellt werden, bei die Formulierung der Hilfsstoffe und/oder der Kapsel eine retardierte Freisetzung bewirken.

Nach Auslassversuchen (drei Tage kein Amifampridin) mit rekurrierender Fatigue Dauermedikation mit einer Tagesdosis von 20 mg bis 30 mg, bevorzugt von 20 bis 25 mg von je 5 mg im Abstand von 4 Stunden, wobei der Bell-Score dauerhaft von 90 bis 100 beträgt. Dieser Bell-Score Wert kann bereits über ein dreiviertel Jahr gehalten werden. Einnahme von morgens bis abends vor dem Schlafengehen.

Der vorgenannte, verbesserte Bell-Score wird vorzugsweise innerhalb weniger als 3 Tagen, weniger als 2 Tagen erreicht. Vorzugsweise innerhalb von 24 Stunden und besonders bevorzugt innerhalb weniger Stunden. Da die Wirkung des mindestens einen Pyridin-3,4-diamin, eines physiologisch verträglichen Salzes, Hydrates, Solvates, Co-Kristalls, Polymorphe dieser und/oder Mischungen dieser, vorzugsweise in der erfindungsgemäßen Zusammensetzung, schnell eintritt, in wenigen Stunden (1, 2, 3, 4, 5 bis 8) oder sofort, nahezu vorzugsweise sofort, deutet eine ausbleibende Wirkung darauf hin, dass eine Dosistitration erforderlich ist, um die angemessene und wirksame Dosis an die Physiologe des Patienten (Gewicht, Größe und/oder Stoffwechselaktivität) anzupassen. Bei angemessener Dosis für den jeweiligen Patienten sollte sich die Wirkung sofort einstellen.

In einem weiteren Aspekt der erfindungsgemäßen Verwendung dauert die Langzeitbehandlung größer gleich zwei Wochen an und kann in eine dauerhafte Behandlung übergehen. In einem weiteren Aspekt der erfindungsgemäßen Verwendung erzielt die Behandlung einen Bell-Score von größer gleich 60, größer gleich 70, größer gleich 80, oder mehr und die Langzeitbehandlung dient zur Aufrechterhaltung eines vordefinierten Bell-Scores. Der vordefinierte Bell-Score entspricht dem individuellen Referenzwert des Patienten vor der viralen Infektion. Alternative entspricht der vordefinierte Bell-Score der als ausreichend oder als angemessen vom Patienten empfundenen Einstufung, die mit dem betreuenden Arzt erfolgt. Daraus ergibt ein Bell-Score, der sich an den beschrieben Merkmalen orientiert. Über den Verlauf der Behandlung, akute Behandlung, Langzeitbehandlung und dauerhafte Behandlung, kann es wünschenswert sein, die Dosis anzupassen, zu erhöhen oder herabzusetzen, um den gewünschten Bell-Score zu erzielen und aufrechtzuerhalten.

Die Bell-score (oder skala) ist ein Massstab der Behinderung bei ME & CFS. Auch für Long Covid-Patient:innen ist sie geeignet. Die Bell-Skala ist individuell, da jeder Patientinnen eine unterschiedliche Belastungsintoleranz aufweist und das Erleiden von schweren Symptomen in Ruhe verhältnismässig unterschiedlich sein kann.
- 100: Keine Symptome in Ruhe; keine Symptome bei körperlicher Belastung; insgesamt ein normales Aktivitätsniveau; ohne Schwierigkeiten in der Lage, Vollzeit zuarbeiten.
- 90: Keine Symptome in Ruhe; leichte Symptome bei körperlicher und geistiger Belastung; insgesamt ein normales Aktivitätsniveau; ohne Schwierigkeiten in der Lage, Vollzeit zu arbeiten.
- 80: Leichte Symptome in Ruhe; die Symptome verstärken sich durch Belastung; nur bei Tätigkeiten, die anstrengend sind, ist eine geringfügige Leistungseinschränkung spürbar; mit Schwierigkeiten in der Lage, an Arbeitsplätzen, die Kraftanstrengungen erfordern, Vollzeit zu arbeiten.
- 70: Leichte Symptome in Ruhe; deutliche Begrenzungen in den täglichen Aktivitäten spürbar; der funktionelle Zustand beträgt insgesamt etwa 90% der Norm - mit Ausnahme von Tätigkeiten, die einer Kraftanstrengung bedürfen; mit Schwierigkeiten in der Lage Vollzeit zu arbeiten.
- 60: Leichte Symptome in Ruhe; deutliche Begrenzungen in den täglichen Aktivitäten spürbar; der funktionelle Zustand beträgt insgesamt etwa 70%-90% der Norm; Unfähig, einer Vollzeitbeschäftigung nachzugehen, wenn dort körperliche Arbeit gefordert wird; aber in der Lage, Vollzeit zu arbeiten, wenn es um leichte Arbeiten geht und die Arbeitszeit flexibel gehandhabt werden kann.
- 50: Mittelschwere Symptome in Ruhe; mittelschwere bis schwere Symptome bei körperlicher Belastung oder Aktivität; der funktionelle Zustand ist auf 70% der Norm reduziert; unfähig, anstrengende Arbeiten durchzuführen, aber in der Lage, leichte Arbeiten oder Schreibtischarbeit für 4-5 Stunden täglich durchzuführen, wobei Ruhepausen benötigt werden.
- 40: Mittelschwere Symptome in Ruhe; mittelschwere bis schwere Symptome bei Belastung oder Aktivität; der funktionelle Zustand ist auf 50%-70% der Norm reduziert; unfähig, anstrengende Arbeiten durchzuführen, aber in der Lage, leichte Arbeiten oder Schreibtischarbeit für 3-4 Stunden täglich durchzuführen, wobei Ruhepausen benötigt werden.
- 30: Mittelschwere bis schwere Symptome in Ruhe; schwere Symptome bei jeglicher Belastung oder Aktivität; der funktionelle Zustand ist auf 50% der Norm reduziert; in der Regel ans Haus gefesselt; unfähig, anstrengende Arbeiten durchzuführen, aber in der Lage, leichte Arbeiten oder Schreibtischarbeit für 2-3 Stunden täglich durchzuführen, wobei Ruhepausen benötigt werden.
- 20: Mittelschwere bis schwere Symptome in Ruhe; schwere Symptome bei jeglicher Belastung oder Aktivität; der funktionelle Zustand ist auf 30%-50% der Norm reduziert; bis auf seltene Ausnahmen unfähig, das Haus zu verlassen; den grössten Teil des Tages ans Bett gefesselt; unfähig, sich mehr als eine Stunde am Tag zu konzentrieren.
- 10: Schwere Symptome in Ruhe; die meiste Zeit bettlägerig; ein Verlassen des Hauses ist nicht möglich; deutliche kognitive Symptome, die eine Konzentration verhindern.
- 0: Ständig schwere Symptome; immer ans Bett gefesselt; unfähig zu einfachsten Pflegemassnahmen.

Das erfindungsgemäße Aminopyridin bewirkt, vorzugsweise in einer entsprechenden Zusammensetzung, eine umgehende Besserung der muskulären Defizite und weiteren Symptomatik, wie die nachstehenden Beispiele es belegen.

### Beispiele

In den nachfolgend genannten Anwendungsbeispielen wurde 3,4-Diaminopyridin Amifamprindin (Formulierung als Fertigarzneimittel) verabreicht. Im Rahmen der doppelt-blind Studie wurden gleich aussehende Kapseln mit Amifampridin oder Placebo apothekenseitig verblindet zubereitet, wobei nur apothekenseitig bekannt war welche Kapseln Wirkstoff oder Placebo enthielten.

Aminopyridin, hier 3,4-Diaminopyridin, hat bei dem in Fall 1 geschilderten Patienten nicht nur eine umgehende Besserung der muskulären Defizite bewirkt, sondern auch eine schlagartige Besserung einer seit Covid-Infektion bestehenden gravierenden Fatigue-Symptomatik mit einem Schlaf- bzw. Ruhebedürfnis bis zu 20 Stunden pro Tag (brain fog) sowie ausgeprägter Konzentrationsschwäche.

Im Fall 2 wird eine Patientin beschrieben, die ebenfalls unter einer gravierenden Fatigue-Symptomatik nach Covid-Infektion 5/22 litt, eine zuzuordnende neurologische oder internistische Grunderkrankung bestand nicht. Es erfolgte die Gabe von 5 mg Amifampridin, darunter war eine komplette Besserung der Fatigue-Symptomatik sowie vollständiger Restitution der Konzentrationsfähigkeit zu konstatieren. Als zuzuordnende Nebenwirkung traten leichte periorale Parästhesien auf, die nicht als störend empfunden wurden. Die nachfolgenden Labor- und EKG-Kontrollen zur Erfassung substanzassoziierter Komplikationen waren unauffällig. Die off-label-Therapie wurde mit einer Tagesdosis von 3 x 5 mg fortgeführt, darunter sistiert die vorbestehende Fatigue vollständig, es besteht wieder vollständige Leistungsfähigkeit.

Im Fall 3 wird ein Patient beschrieben, der nach zweimaliger Covid-Infektion unter Fatigue-Symptomatik mit einem Schlafbedürfnis bis zu 2 x 9 Stunden pro Tag litt. Auch hier erfolgte ein off-label-Therapieversuch mit 5 mg Amifampridin, die ebenso wie in den zuvor beschriebenen Fällen zu einer schlagartigen Besserung der Konzentrationsfähigkeit sowie physiologischem Schlaf- und Ruhebedürfnis führte. Auch hier wurden transiente periorale Parästhesien mit sehr geringer Intensität bemerkt.

Nach den bisherigen klinischen Beobachtungen besteht offensichtlich ein gute Wirksamkeit des Amifampridin bei Fatigue-Symptomatik ohne Vorliegen einer neurologischen Grunderkrankung, die in dieser Form bisher nicht beschrieben wurde.

Es ist bekannt, dass Amifampridin (Diaminpyridin) bei LEMS-Patienten zu einer Besserung einer koindizierten Fatigue von LEMS-Patienten führt, wobei aufgrund der niedrigen Inzidenz von LEMS-Patienten (1 : 100.000) keine dahingehenden Studien vorliegen. Für das chemisch eng verwandte Fampridin (Monoaminpyridin), das bei MS-Patienten verwendet wird, ist in der Literatur die Besserung einer begleitenden Fatigue-Symptomatik beschrieben. Ob es sich bei den beschriebenen positiven Einflüssen auf eine Fatigue-Symptomatik bei der Aminpyridine um einen substanzspezifischen Effekt im Rahmen der neurologischen Grunderkrankung handelt, ist bislang nicht hinreichend geklärt. Aufgrund der geschilderten Fälle ist ein unspezifischer positiver Effekt auf die Fatigue anzunehmen, der durch weitere Forschung noch geklärt werden muss, aus den hier vorgestellten Falldarstellungen ist jedoch eine Wirksamkeit bei Fatigue-Symptomatik im Rahmen eines Long-Covid-Syndroms abzuleiten.

Das Lambert-Eaton-Myasthenes-Syndrom (LEMS) ist eine seltene Erkrankung. Häufig tritt sie im Rahmen einer Tumorerkrankung (SCLC) oder seltener im Rahmen von Autoimmunerkrankungen auf. LEMS mit kleinzelligem Lungenkarzinom wird als SCLC-LEMS und das nichttumoröse LEMS als NT-LEMS bezeichnet. Bei 50 bis 60 % der Patienten wird SCLC-LEMS gemeinsam mit einem aufgrund von Rauchen assoziierten, neuroendokrinen Tumor festgestellt. Einige wenige Berichte beschreiben LEMS auch im Zusammenhang mit anderen Tumoren. Diese Tumore weisen dann häufig einen neuroendokrinen Charakter auf. Beispiele dafür sind anderen Lungentumore oder Prostatakarzinom, also dem häufigsten extrathorakalen neuroendokrinen Tumor, der häufig mit einem Anti-Hu-Syndrom verbunden ist.

Bei LEMS handelt es sich um eine Störung der Impulsübertragung zwischen Nerv und Muskel und dadurch kommt es zu einer Schwäche der Muskulatur. Die Muskelschwäche bei LEMS wird durch eine fehlgesteuerte Immunreaktion ausgelöst. Denn es werden Antikörper gegen körpereigene, spannungsabhängige Kalziumkanäle vom P/Q-Typ gebildet, die sich an der Impulsübertragungs-stelle (Synapse) befinden. Die Antikörperbildung vermindert die Freisetzung des Botenstoffs Acetlycholin an den Synapsen des motorischen und autonomen Nervensystems und stört somit die Übertragungsleitung.

Bei LEMS handelt sich daher um eine präsynaptische, neuromuskuläre Impulsübertragungsstörung. Wie vorstehend erläutert, steht das Lambert-Eaton-Myasthenes- Syndrom häufig im Zusammenhang mit einer Tumorerkrankung (paraneoplastisches LEMS). LEMS kann der Tumordiagnose hierbei zwei bis maximal fünf Jahre vorausgehen, und zur Früherkennung des Tumors beitragen. Die häufigste Krebserkrankung ist hierbei in der Lunge das kleinzellige Bronchialkarzinom, für das Rauchen als Auslöser bewiesen ist. Aber auch andere Tumore der Lunge, der Prostata, des Thymus oder andere Organe können mit einem LEMS assoziiert sein. Gleichfalls kann LEMS als eine eigenständige Autoimmunerkrankung unabhängig von Tumorerkrankungen auftreten. LEMS ist eine seltene Erkrankung mit 1 Erkrankten auf 250.000 bis 300.000 Personen.

In weiteren Studien werden verschiedenen Konzentrationen, Tagesdosens, sowie Anzahl der Dosen untersucht in Relations zum Bell-Score vor und nach der Behandlung über einen definierten Zeitraum untersucht.

Dazu werden/können ergänzende Parameter herangezogen, die eben geeignet sind die Symptome wie Schlafstörungen, Müdigkeit, erhöhtes Schlafbedürfnis, muskuläre Schwäche, verringerte körperliche Belastbarkeit, verringerte psychische Belastbarkeit, verringertes Erinnerungsvermögen, Denk- und Merkbeeinträchtigung, Konzentrationsverlust, erhöhtes Schmerzempfinden und/oder ein Aufmerksamkeitsdefizit zu qualifizieren und zu quantifizieren. Hierzu können biochemischen Parametern kombiniert werden, welche zur Analyse von Kreislauf, Hormon- und/oder Immunsystem geeignet sind, umfassen die Messung der Hormone u.a. Melatonin, Cortisol und/oder Serotonin, die Messung des oberen, systolischen und des unteren, diastolischen Wertes, Bestimmung des Blutzuckers usw.

Es wurde eine weitere Studie an einer Patientengruppe durchgeführt. Auch in dieser Studie konnte eine reduzierte Fatigue-Symptomatik bei Post-Covid-Syndrom unter Amifampridin festgestellt werden.

Die Prävalenz eines Post-Covid-Syndroms (PCS) liegt bei 10-15 % nach SARS-COV-2-Infektion, neben spezifischen Organerkrankungen treten vielfältige funktionelle Beschwerden auf, vorrangig eine Fatigue-Symptomatik. Diese ist gekennzeichnet durch persistierende Müdigkeit, Schwäche, kognitive Defizite sowie vermehrtem Schlafbedürfnis. In dieser Studie bzw. Fallserie wird der Effekt von Amifampridin bei Patienten mit PCS beschrieben, die unter unphysiologisch gesteigertem Schlafbedürfnis sowie eingeschränkter Kognition litten. Zur Evaluation wurde der Bell-Score verwendet, der auf die Einschränkung der Vigilanz, körperlichen Belastbarkeit und Berufstätigkeit fokussiert.

Bei Pat. 1 der weiteren Studie ist vorgeschichtlich eine Zn. EBV-Infektion bekannt, ansonsten keine internistischen oder neurologischen Vorerkrankungen. Nach SARS-COV-2-Infektion im Mai 2022 fluktuierende Fatigue-Symptomatik mit einem Bell-Score zwischen 20 und 40, gesteigertes Schlafbedürfnis mit 12 Stunden / 24 h. Nach dreimaliger Einnahme von 5 mg Amifampridin über 24 h (h: Stunden) bestand vollständige Rekonvaleszenz mit einem Bell-Score von 100, das Schlafbedürfnis reduzierte sich auf 7 Stunden / 24h. Auslassversuche mündeten in einer erneuten Fatigue.

Bei Pat. 2, der weiteren Studie, einem 60jährigen Patienten trat nach zweimaliger SARs-Cov-2-Infektion eine schwere Fatigue-Symptomatik mit gravierender Einschränkung der beruflichen Tätigkeit auf. Bell-Score 40, das Schlafbedürfnis lag bei 12 Stunden / 24h, in den Wachphasen eingeschränkte Vigilanz. Nach dreimaliger Gabe von 5 mg Amifampridin wurde ein Bell-Score 100 erreicht, das Schlafbedürfnis sank auf 7,5 Stunden /24 h, uneingeschränkte Vigilanz. Gleiche Effekte bei rekurrierender Fatigue.

Bei Pat. 3, der weiteren Studie, einer 26jährigen Patientin, bestand nach zweimaliger SARS-COV-2-Infektion 2022 eingeschränkte Konzentrationsfähigkeit, Schlafbedürfnis 11 bis 12 Stunden / 24 h, Bell-Score 40. Nach einer spontanen Einnahme von 5 mg Amifampridin erfolgte eine Reduktion des Schlafbedürfnisses auf 7-9 Stunden, Bell-Score 100. Der Effekt hielt etwa 36 Stunden an und war durch erneute Einnahme von Amifampridin reproduzierbar.

Pat. 4, der weiteren Studie, einer 36 Jahre alten Patientin, betreute während der ersten Covid-Welle schwerst Erkrankte auf einer Intensivstation, kurz danach typische Symptomatik mit Geschmacksverlust und Post-Covid-Myokarditis. Ausgeprägte Einschränkung sowohl der körperlichen Belastbarkeit (Bell-Score 20) sowie gesteigertes Schlafbedürfnis auf 12 bis 14 Stunden (h) / 24 Stunden (h). Nach dreimaliger Einnahme von je 5 mg Amifampridin über 24 h als Tagesdosis ist Fatigue-Symptomatik rückläufig und muskuläre Beschwerden (Bell-Score 50) verbessern sich sowie sinkt das Schlafbedürfnis auf 8 bis 10 Stunden / 24h. Der Effekt war reproduzierbar. Die noch bestehende Einschränkung der Belastbarkeit ist durch die kardiale Problematik bedingt.

Bei dem 63-jährigen Pat. 5, der weiteren Studie, bestand seit 2021 eine neuromuskuläre Erkrankung (Lambert-Eaton-Myasthenie-Syndrom synonym zu Lambert-Eaton-Myasthenes-Syndrom), nach SARS-COV-2-Infektion 5/22 schwere Fatigue, Schlafbedürfnis 16 bis 18 Stunden /24h, Bell-Score 10. Nach drei oder viermaliger Gabe von 5 mg Amifampridin als Tagesdosis sistierte die Fatigue, Schlafbedürfnis 8-9 Stunden / 24h, gebesserte muskuläre Belastbarkeit.

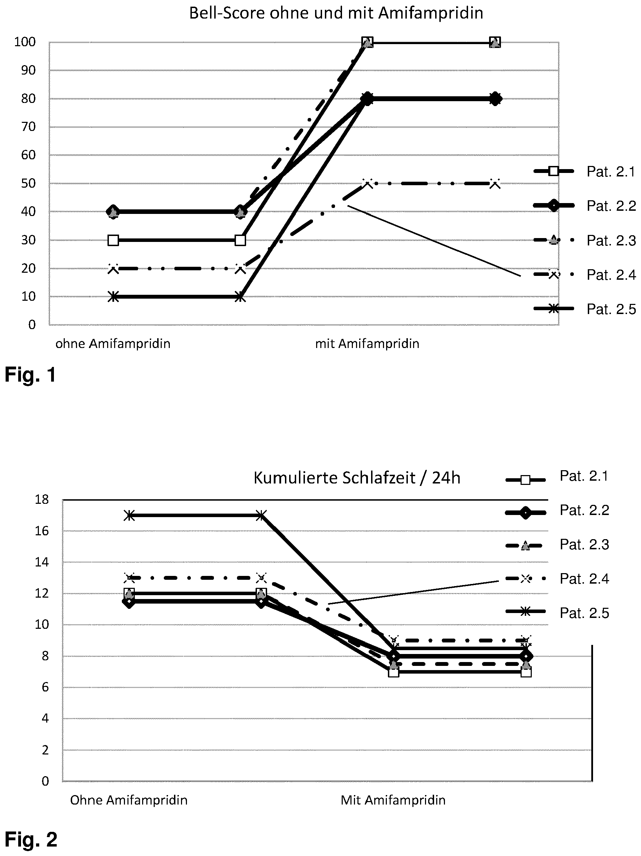

Die nachfolgenden Dosierungen der Patienten 2.1 bis 2.5 und die veränderten Bell-Sore Werte sowie das Schlafbedürfnis in Stunden sind in den Figuren 1 und 2 dargestellt (**Fig. 1** Bell-Score mit und ohne Amifampridin, Patienten 2.1 bis 2.5, **Fig. 2****:** kumulierte Schlafzeit in 24 h).

Bei Pat. 2.1, einer 61jährigen Psychotherapeutin ohne internistischen oder neurologischen Vorerkrankungen trat nach SARS-COV-2-Infektion im Mai 2022 fluktuierende Fatigue-Symptomatik mit einem Bell-Score zwischen 20 und 40 sowie gesteigertes Schlafbedürfnis mit 12 Stunden / 24 h auf. Nach einmaliger Einnahme von 5 mg Amifampridin bestand vollständige Rekonvaleszenz mit einem Bell-Score von 100, das Schlafbedürfnis reduzierte sich auf 7 Stunden / 24h.

Nach Auslassversuchen (drei Tage kein Amifampridin) mit rekurrierender Fatigue erfolgte eine Dauermedikation mit einer Tagesdosis von 20 mg bis 25 mg von je 5 mg im Abstand von 4 Stunden, wobei der Bell-Score dauerhaft von 90 bis 100 beträgt. Dieser Bell-Score Wert kann bereits über ein dreiviertel Jahr gehalten werden. Einnahme von morgens bis abends vor dem Schlafengehen.

Bei Pat. 2.2, einer 26jährigen Medizinstudentin, bestand nach zweimaliger SARS-COV-2-Infektion 2022 eingeschränkte Konzentrationsfähigkeit, Schlafbedürfnis 11-12 Stunden / 24 h, Bell-Score 40. Nach Einnahme von 5 mg Amifampridin Reduktion des Schlafbedürfnisses auf 7-9 Stunden, Bell-Score 80. Der Effekt hielt etwa 36 Stunden an und war durch erneute Einnahme von Amifampridin reproduzierbar, anschließende Dauermedikation mit einer Tagesdosis von 20 mg bis 25 mg von je 5 mg im Abstand von 4 Stunden, wobei der Bell-Score dauerhaft von 90 bis 100 beträgt. Dieser Wert kann bereits seit einem Monat reproduziert werden.

Bei Pat. 2.3, einem 60jährigen Rechtsanwalt, trat nach zweimaliger SARS-COV-2-Infektion eine schwere Fatigue-Symptomatik auf. Bell-Score 40, das Schlafbedürfnis lag bei 12 Stunden / 24h, in den Wachphasen eingeschränkte Vigilanz. Nach 5 mg Amifampridin Bell-Score 100, Schlafbedürfnis 7,5 Stunden /24 h, uneingeschränkte Vigilanz. Gleiche Effekte bei rekurrierender Fatigue, bei weiterer bedarfsweiser Einnahme von 5 mg Amifampridin.

Die 36jährige Patientin 2.4 betreute während der ersten Covid-Welle schwerst Erkrankte auf Intensivstation, kurz danach typische Symptomatik mit Geschmacksverlust, muskuläre Beschwerde und Post-Covid-Myokarditis. Ausgeprägte Einschränkung sowohl der körperlichen Belastbarkeit (Bell-Score 20) sowie gesteigertes Schlafbedürfnis auf 12-14 Stunden / 24 h. Nach 5 mg Amifampridin rückläufige Fatigue-Symptomatik und muskuläre Beschwerden (Bell-Score 50) sowie Verminderung des Schlafbedürfnisses auf 8 bis 10 Stunden / 24h. Der Effekt war reproduzierbar. Bei Bedarf nimmt diese Patientin teilweise im Abstand von Wochen jeweils einzeln 5 mg Amifampridin, um den Bell-Score 60 und die Fatigue-Symptomatik zu verbessern.

Die noch bestehende Einschränkung der Belastbarkeit ist durch die kardiale Problematik bedingt.

Pat. 2.5: 63jähriger Kardiologe bestand seit 2021 eine neuromuskuläre Erkrankung (Lambert-Eaton-Myasthenie-Syndrom), nach SARS-COV-2-Infektion 5/22 schwere Fatigue, Schlafbedürfnis 16-18 Stunden /24h, Bell-Score 10. Nach einer initialen Gabe 3 x 5 mg Amifampridin sistierte die Fatigue, Schlafbedürfnis 8-9 Stunden / 24h, gebesserte muskuläre Belastbarkeit, Bell-Score 80. Trotz Fortschreitender LEMS weiterhin gebesserte Fatigue. Über einen Zeitraum etwa 9 Monaten wurden bis zu 60 mg und seit zwei Wochen 70 mg Amifampridin mit der Grunderkrankung LEMS eingenommen. Zu Beginn konnte eine Verbesserung des Bell-Score bereits mit 15 mg am Tag erzielt werden. Im Laufe der vorgenannten Monate wurde die Dosis auf 60 mg und später auf 70 mg am Tag erhöht. Zunächst wurde mit 3 mal 5 mg Tagesdosis eine Verbesserung erzielt. Anschließend betrug die Einzeldosis je 3 mal 20 mg und ggf. zusätzlich einer Einzeldosis von 5 mg. Die Abstände zwischen den 20 mg Dosen liegen vom Aufstehen bis zum Zubettgehen annähernd gleichmäßig verteilt bei ca. 6 bis 7 Stunden und bei Bedarf zusätzlich weitere 5 mg Amifampridin. Die Fatigue-Symptomatik ist dadurch zufriedenstellend kontrolliert Bell-Score 70 bis 80 in Bezug auf Fatigue und in Bezug auf die muskulären Defizite ein Bell-Score von ca. 20. Vor einer Gabe vom Amifampridin lag der Bell-Score bei 20 bis 30 in Bezug auf die Fatigue und Gesamtzustand.

Bei Pat. 2.1 und 2.2 waren zur Rekonvaleszenz eine Dauermedikation mit jeweils 20 mg Amifampridin pro Tag erforderlich und boten damit Voraussetzungen für einen doppelt-blinden Auslassversuch über jeweils 3 Tage.

Pat. 2.1 mit Verum an Tagen 1-3, nachfolgend Placebo an den Tagen 4-6:
Pat. (Pat. 2.1) mit Bell-Score 90 begann bereits mit Amifampridin, wie vorstehend beschrieben, und setzte diese mit Blister mit 6 Tagesdosen je Tag ein Fach mit Kapseln für 3 Tage mit je 4 mal 5 mg Amifampridin fort. Die Dosierung betrug jeweils für 3 Tage Placebo mit je 4mal Placebo-Kapseln für Patient 1 und 2 (Verum und Placebo).

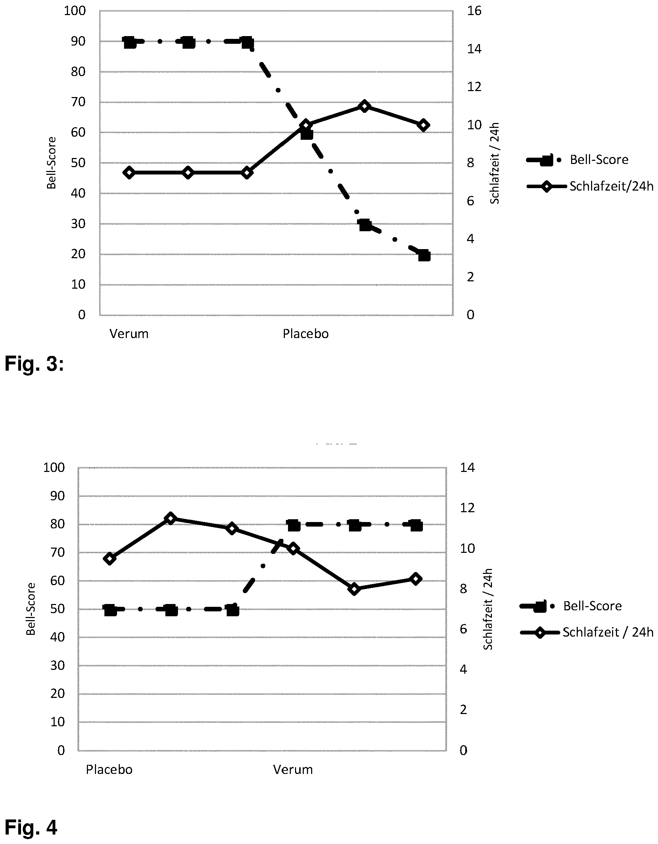

Pat. 2.1: Verum an Tagen 1-3, nachfolgend Placebo an den Tagen 4-6: In der Placebophase Reduktion des Bell-Score auf minimal 30, gesteigerte Schlafzeit auf 10,5 Stunden (**Fig. 3****:** Pat. 2.1, Bell-Score (Linie mit Quadraten mit Seitenlinien der Quadrate parallel und senkrecht zur Hauptlinie; Schlafzeit/24 h (Linie mit kleineren Quadraten, Spitzen Quadrate zeigen nach oben/unten sowie rechts und links).

Pat.2.2: Placebo an den Tagen 1-3, Verum an den Tagen 4-6: Reduktion des Bell-Score unter Placebo auf 50, gesteigerte Schlafzeit auf 11 Stunden (**Fig. 4****:** Pat. 2.2, Bell-Score (Linie mit Quadrat, Seitenlinie parallel zur Hauptlinie, Schlafzeit/24 h (Linie mit kleinerem Quadrat, Spitzen zeigen nach oben/unten sowie rechts und links).

Geplant sind weitere Doppeltblind Studien mit Patienten mit einer Initialdosis: von 3 x 5 mg Amifampridin und bei Nicht-Wirken mit einer Dosis von 3 x 10 mg an Amifampridin über eine Dauer von zwei Wochen. Die Kontrollgruppe erhält ein Placebo.

Fatigue ist ein Begleitphänomen bei internistischen, neurologischen und onkologischen Erkrankungen mit erheblichem Verlust der Lebensqualität. Bei zunehmender Inzidenz von PCS bestehen gehäufte Arbeits- und Erwerbsunfähigkeiten infolge Fatigue.

Die molekularen Mechanismen sind noch nicht entschlüsselt, hinsichtlich symptomatischer Therapieansätze sind günstige Effekte des Fampridin (Monoaminopyridin) auf Fatigue in der Therapie der Multiplen Sklerose bekannt. Seitens des Universitätsklinikums Basel wird hierzu eine Studie zur Frage des Einflusses von Fampridin auf das Arbeitsgedächtnis durchgeführt. Amifampridin ist als orphan drug singulär für das Lambert-Eaton-Myasthenie-Syndrom wie bei Pat. 5 indiziert. Besserung einer koinzidenten Fatigue als Phosphate mit LEMS sind beschrieben für 20 bis 80 mg als Tagesdosen (Clinical Trial J Clin Neuromuscul Dis. 2019 Mar;20(3):111-119: Shieh, Perry; Sharma, Khema, Kohrman, Bruce; Oh, Shin J: Amifampridine Phosphate (Firdapse) Is Effective in a Confirmatory Phase 3 Clinical Trial in LEMS). In allen beschriebenen erfindungsgemäßen Fällen bestand nach niedrig-dosierter Gabe von, insbesondere einmaliger bis viermaliger Gabe von 5 mg als Tagesdosis, bevorzugt von einmaliger bis dreimaliger Tagesdosis, eine signifikante Reduktion der durch eine virale Infektion durch ss(+)RNA-Viren der Familie Coronavirdidae ausgelösten Fatigue sowie des durch diese Viren ausgelösten unphysiologisch gesteigerten Schlafbedürfnisses, die Effekte waren in Auslassversuchen reproduzierbar. Weitere kontrollierte Studien sind erforderlich.

## Patentansprüche

1. Pyridin-3,4-diamin, ein physiologisch verträgliches Salz, Solvat, Hydrat, Co-Kristall oder Polymorph davon und/oder Gemische enthaltend zwei der genannten Verbindungen zur Verwendung zur Prophylaxe und/oder Behandlung einer Fatigue, welche mit einer viralen Infektion assoziiert ist und, worin die virale Infektion durch ss(+)RNA-Viren der Familie Coronaviridae umfassend das Virus SARS-CoV-1, SARS-CoV-2, MERS-CoV, HCoV-HKU1, HCoV-OC43, HCoV-NL63 und/oder HCoV-229E ausgelöst wird.

2. Pyridin-3,4-diamin zur Verwendung nach Anspruch 1, i) zur Prophylaxe und/oder Behandlung einer komplexen neuroimmunologischen Erkrankung umfassend Myalgische Enzephalomyelitis und/oder ii) eines Chronischen Fatigue Syndroms (ME/CFS), bevorzugt eines postinfektiösen Erschöpfungssyndroms.

3. Pyridin-3,4-diamin zur Verwendung nach Anspruch 1, worin die virale Infektion eine SARS-CoV-2-Infektion ist.

4. Pyridin-3,4-diamin zur Verwendung nach einem der Ansprüche 1 bis 3, worin die Fatigue eine postinfektiöse Fatigue ist, vorzugsweise nach Abklingen der viralen Infektion auftritt, vorzugsweise nach Feststellen eines Ct-Wertes von größer gleich 30 und optional i) einer komplexen neuroimmunologischen Erkrankung umfassend myalgische Enzephalomyelitis und/oder optional ii) Chronisches Fatigue Syndrom (ME/CFS), bevorzugt eines postinfektiösen Erschöpfungssyndroms.

5. Pyridin-3,4-diamin zur Verwendung nach einem der Ansprüche 1 bis 4, worin die gesundheitlichen Beschwerden der akuten Krankheitsphase einer viralen, vorzugweise SARS-CoV-2, Infektion, über größer gleich 3, größer gleich 4 Wochen fortbestehen oder neu auftreten.

6. Pyridin-3,4-diamin zur Verwendung nach einem der Ansprüche 1 bis 5, worin die Fatigue Symptome ausgewählt aus Schlafstörungen, Müdigkeit, erhöhtes Schlafbedürfnis, muskuläre Schwäche, verringerte körperliche Belastbarkeit, verringerte psychische Belastbarkeit, verringertes Erinnerungsvermögen, Denk- und Merkbeeinträchtigung, Konzentrationsverlust, erhöhtes Schmerzempfinden und/oder ein Aufmerksamkeitsdefizit und/oder Fehlregulationen von Kreislauf, Hormon- und/oder des Immunsystem umfasst.

7. Pyridin-3,4-diamin zur Verwendung nach einem der Ansprüche 1 bis 6, worin die Fatigue bei einem Bell-Score von kleiner gleich 50, kleiner gleich 40, kleiner gleich 30, kleiner gleich 20 vorliegt.

8. Pyridin-3,4-diamin zur Verwendung nach einem der Ansprüche 1 bis 7, worin der Schlafbedarf auf physiologische Stunden größer gleich 6 bis kleiner gleich 12 reduziert wird, vorzugsweise größer gleich 6, größer gleich 7, größer gleich 8 bis kleiner gleich 11 Stunden, kleiner gleich 10, kleiner gleich 9, besonders bevorzugt größer gleich 6 bis kleiner 8.

9. Pyridin-3,4-diamin zur Verwendung nach einem der Ansprüche 1 bis 8, worin eine Dosis von größer gleich 1 mg, größer gleich 2 mg, größer gleich 3 mg, vorzugsweise größer gleich 5 mg bis kleiner gleich 60 mg pro Tag verabreicht wird.

10. Pyridin-3,4-diamin zur Verwendung nach einem der Ansprüche 1 bis 9, worin eine maximale Tagesdosis kleiner gleich 60 mg aufweist, vorzugsweise größer gleich eine bis kleiner gleich fünf Dosen pro Tag verabreicht werden, besonders bevorzugt enthält eine Dosis größer gleich 2, größer gleich 2,5 größer gleich 3 mg, größer gleich 5 mg bis kleiner gleich 20 mg, bis kleiner gleich 18 mg, vorzugsweise bis kleiner gleich 15 mg, jeweils des wirksamen Pyridin-3,4-diamin, eines physiologisch verträglichen Salzes, Solvates, Hydrates, Co-Kristalls oder Polymorphs davon und/oder Gemische enthaltend zwei der genannten Verbindungen.

11. Pyridin-3,4-diamin zur Verwendung nach einem der Ansprüche 1 bis 10, wobei die Behandlung eine Langzeitbehandlung mit einer wirksamen Dosis ist, vorzugsweise ist die wirksame Dosis auf die Physiologie des Patienten angepasst und auf das Erreichen eines Bell-Scores von größer gleich 60, größer gleich 70, größer gleich 80, größer gleich 90 bis zu 100 und/oder ein Kraftgrad von mindestens 4 ausgerichtet.

12. Pyridin-3,4-diamin zur Verwendung nach einem der Ansprüche 1 bis 11, wobei die Langzeitbehandlung größer gleich zwei Wochen andauert und optional in eine dauerhafte Behandlung übergeht.

13. Pyridin-3,4-diamin zur Verwendung nach einem der Ansprüche 1 bis 12, wobei die Behandlung einen Bell-Score von größer gleich 60, größer gleich 70 größer gleich 80 oder mehr erzielt und die Langzeitbehandlung zur Aufrechterhaltung des Bell-Scores dient.

14. Pharmazeutische Zusammensetzung umfassend Pyridin-3,4-diamin, ein physiologisch verträgliches Salz, Solvat, Hydrat, Co-Kristall oder Polymorph davon und/oder Gemische enthaltend zwei der genannten Verbindungen sowie mindestens einen pharmazeutischen Hilfsstoff zur Verwendung zur Prophylaxe und/oder Behandlung einer Fatigue nach einem der Ansprüche 1 bis 13.

## Claims

1. Pyridine-3,4-diamine, a physiologically tolerable salt, solvate, hydrate, co-crystal, polymorphs thereof and/or mixtures containing two of the mentioned compounds for the use for the prophylaxis and/or treatment of a fatigue, which is associated with a viral infection and wherein the viral infection is triggered by ss(+)RNA viruses of the family Coronaviridae comprising the virus SARS-CoV-1, SARS-CoV-2, MERS-CoV, HCoV-HKU1, HCoV-OC43, HCoV-NL63 and/or HCoV-229E.

2. Pyridine-3,4-diamine for the use according to claim 1, i) for the prophylaxis and/or treatment of a complex neuroimmunological disease comprising myalgic encephalomyelitis and/or optionally ii) of a chronic fatigue syndrome (ME/CFS), preferably of a post-infectious fatigue syndrome.

3. Pyridine-3,4-diamine for the use according to claim 1, wherein the viral infection is a SARS-CoV-2 infection.

4. Pyridine-3,4-diamine for the use according to any one of claims 1 to 3, wherein the fatigue is a post-infectious fatigue, preferably appears after abatement of the viral infection, preferably after determination of a Ct value of greater than or equal to 30 and optionally i) of a complex neuroimmunological disease comprising myalgic encephalomyelitis and/or optionally ii) chronic fatigue syndrome (ME/CFS), preferably of a post-infectious fatigue syndrome.

5. Pyridine-3,4-diamine for the use according to any one of claims 1 to 4, wherein the health problems of the acute disease phase of a viral, preferably SARS-CoV-2, infection, persist for greater than or equal to 3, greater than or equal to 4 weeks or appear again.

6. Pyridine-3,4-diamine for the use according to any one of claims 1 to 5, wherein the fatigue comprises symptoms selected from sleep disorders, tiredness, increased need for sleep, muscular weakness, decreased physical endurance, decreased mental endurance, decreased recollection ability, impairment in the ability to think and remember, loss of concentration, increased pain perception and/or an attention deficit and/or dysregulations of the circulation, hormone and/or of the immune system.

7. Pyridine-3,4-diamine for the use according to any one of claims 1 to 6, wherein the fatigue is present with a Bell score of less than or equal to 50, less than or equal to 40, less than or equal to 30, less than or equal to 20.

8. Pyridine-3,4-diamine for the use according to any one of claims 1 to 7, wherein the need for sleep is reduced to physiological hours of greater than or equal to 6 to less than or equal to 12, preferably greater than or equal to 6, greater than or equal to 7, greater than or equal to 8 to less than or equal to 11 hours, less than or equal to 10, less than or equal to 9, particularly preferably greater than or equal to 6 to less than 8.

9. Pyridine-3,4-diamine for the use according to any one of claims 1 to 8, wherein a dose of greater than or equal to 1 mg, greater than or equal to 2 mg, greater than or equal to 3 mg, preferably greater than or equal to 5 mg to less than or equal to 60 mg per day is administered.

10. Pyridine-3,4-diamine for the use according to any one of claims 1 to 9, wherein a maximum daily dose has less than or equal to 60 mg, preferably greater than or equal to one to less than or equal to five doses per day are administered, a dose particularly preferably contains greater than or equal to 2, greater than or equal to 2.5 greater than or equal to 3 mg, greater than or equal to 5 mg to less than or equal to 20 mg, to less than or equal to 18 mg, preferably to less than or equal to 15 mg, of the at least one effective Pyridine-3,4-diamine each, a physiologically tolerable salt, solvate, hydrate, co-crystal, polymorphs thereof and/or mixtures containing two of the mentioned compounds.

11. Pyridine-3,4-diamine for the use according to any one of claims 1 to 10, wherein the treatment is a long-term treatment with an effective dose, the effective dose is preferably adapted to the physiology of the patient and is geared towards reaching a Bell score of greater than or equal to 60, greater than or equal to 70, greater than or equal to 80, greater than or equal to 90 up to 100 and/or a strength score of at least 4.

12. Pyridine-3,4-diamine for the use according to any one of claims 1 to 11, wherein the long-term treatment lasts greater than or equal to two weeks and optionally transitions into a permanent treatment.

13. Pyridine-3,4-diamine for the use according to any one of claims 1 to 12, wherein the treatment achieves a Bell score of greater than or equal to 60, greater than or equal to 70 greater than or equal to 80 or more and the long-term treatment serves the purpose of maintaining the Bell score.

14. A pharmaceutical composition comprising Pyridine-3,4-diamine, a physiologically tolerable salt, solvate, hydrate, co-crystal, polymorphs thereof and/or mixtures containing two of the mentioned compounds as well as at least one pharmaceutical excipient for the use for the prophylaxis and/or treatment of a fatigue according to any of the claims 1 to 13.

## Revendications

1. La pyridine-3,4-diamine, un sel physiologiquement tolérable, un solvate, un hydrate, un co-cristal ou un polymorphe de ceux-ci et/ou des mélanges contenant deux desdits composés pour une utilisation dans la prophylaxie et/ou le traitement d'une fatigue associée à une infection virale et l'infection virale étant provoquée par des virus à ARN simple brin (+) de la famille des Coronaviridae comprenant les virus SARS-CoV-1, SARS-CoV-2, MERS-CoV, HCoV-HKU1, HCoV-OC43, HCoV-NL63 et/ou HCoV-229E.

2. La pyridine-3,4-diamine pour une utilisation selon la revendication 1, i) pour la prophylaxie et/ou le traitement d'une maladie neuro-immunologique complexe comprenant l'encéphalomyélite myalgique et/ou ii) un syndrome de fatigue chronique (ME/CFS), préférablement un syndrome de fatigue post-infectieuse.

3. La pyridine-3,4-diamine pour une utilisation selon la revendication 1, l'infection virale étant une infection par le SRAS-CoV-2.

4. La pyridine-3,4-diamine pour une utilisation selon l'une des revendications 1 à 3, la fatigue étant une fatigue post-infectieuse, survenant de préférence après la disparition de l'infection virale, de préférence après la détermination d'une valeur de Ct supérieure ou égale à 30, et éventuellement i) une maladie neuro-immunologique complexe comprenant une encéphalomyélite myalgique et/ou éventuellement ii) un syndrome de fatigue chronique (ME/CFS), préférablement un syndrome de fatigue post-infectieux.

5. La pyridine-3,4-diamine pour une utilisation selon l'une des revendications 1 à 4, les troubles de santé de la phase aiguë d'une infection virale, de préférence SARS-CoV-2, persistant ou réapparaissant pendant une durée supérieure ou égale à 3, supérieure ou égale à 4 semaines.

6. La pyridine-3,4-diamine pour une utilisation selon l'une des revendications 1 à 5, la fatigue comprenant des symptômes choisis parmi les troubles du sommeil, la fatigue, le besoin accru de sommeil, la faiblesse musculaire, la diminution de la résistance physique, la diminution de la résistance psychologique, la diminution de la mémoire, l'altération de la pensée et de la mémoire, la perte de concentration, l'augmentation de la sensibilité à la douleur et/ou un déficit de l'attention et/ou des dérégulations des systèmes circulatoire, hormonal et/ou immunitaire.

7. La pyridine-3,4-diamine pour une utilisation selon l'une des revendications 1 à 6, la fatigue étant présente à un score de Bell inférieur ou égal à 50, inférieur ou égal à 40, inférieur ou égal à 30, inférieur ou égal à 20.

8. La pyridine-3,4-diamine pour une utilisation selon l'une des revendications 1 à 7, le besoin de sommeil étant réduit à des heures physiologiques supérieures ou égales à 6 jusqu'à inférieures ou égales à 12, préférablement supérieures ou égales à 6, supérieures ou égales à 7, supérieures ou égales à 8 jusqu'à inférieures ou égales à 11 heures, inférieures ou égales à 10, inférieures ou égales à 9, plus préférablement supérieures ou égales à 6 jusqu'à inférieures à 8.

9. La pyridine-3,4-diamine pour une utilisation selon l'une des revendications 1 à 8, une dose supérieure ou égale à 1 mg, supérieure ou égale à 2 mg, supérieure ou égale à 3 mg, de préférence supérieure ou égale à 5 mg et inférieure ou égale à 60 mg étant administrée par jour.

10. La pyridine-3,4-diamine pour une utilisation selon l'une des revendications 1 à 9, une dose journalière maximale étant inférieure ou égale à 60 mg, de préférence supérieure ou égale à une dose jusqu'à inférieure ou égale à cinq doses par jour, contenant plus préférablement une dose supérieure ou égale à 2, supérieure ou égale à 2,5, supérieure ou égale à 3 mg, supérieure ou égale à 5 mg jusqu'à inférieure ou égale à 20 mg, jusqu'à inférieure ou égale à 18 mg, de préférence jusqu'à inférieure ou égale à 15 mg, respectivement de pyridine-3,4-diamine active, d'un sel physiologiquement tolérable, d'un solvate, d'un hydrate, d'un co-cristal ou d'un polymorphe de ceux-ci et/ou de mélanges contenant deux des composés mentionnés.

11. La pyridine-3,4-diamine pour une utilisation selon l'une des revendications 1 à 10, le traitement étant un traitement de longue durée avec une dose efficace, de préférence la dose efficace étant adaptée à la physiologie du patient et visant à atteindre un score de Bell supérieur ou égal à 60, supérieur ou égal à 70, supérieur ou égal à 80, supérieur ou égal à 90 jusqu'à 100 et/ou un degré de force d'au moins 4.

12. La pyridine-3,4-diamine pour une utilisation selon l'une des revendications 1 à 11, le traitement de longue durée étant supérieur ou égal à deux semaines et se transformant éventuellement en un traitement permanent.

13. La pyridine-3,4-diamine pour une utilisation selon l'une des revendications 1 à 12, le traitement permettant d'obtenir un score de Bell supérieur ou égal à 60, supérieur ou égal à 70, supérieur ou égal à 80, ou plus, et le traitement de longue durée servant à maintenir le score de Bell.

14. Composition pharmaceutique comprenant la pyridine-3,4-diamine, un sel physiologiquement tolérable, un solvate, un hydrate, un co-cristal ou un polymorphe de ceux-ci et/ou des mélanges contenant deux des composés mentionnés ainsi qu'au moins un excipient pharmaceutique pour une utilisation dans la prophylaxie et/ou le traitement d'une fatigue selon l'une des revendications 1 à 13.
